(19) Europäisches Patentamt
European Patent Office
Office européen des brevets

(11) **EP 3 769 719 A1**

(12) **EUROPEAN PATENT APPLICATION**

(43) Date of publication:
**27.01.2021 Bulletin 2021/04**

(51) Int Cl.:
*A61F 2/12* *(2006.01)*    *A61M 5/142* *(2006.01)*

(21) Application number: **19187842.0**

(22) Date of filing: **23.07.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **National University of Ireland Galway**
  **Galway (IE)**
• **Massachusetts Institute of Technology**
  **Cambridge, MA 02139 (US)**

(72) Inventors:
• **Roche, Ellen**
  **Cambridge, MA 02141 (US)**

• **Dolan, Eimear**
  **Hollymount, Co. Mayo (IE)**
• **Duffy, Garry**
  **Kilcolgan, Co. Galway H91 DVF7 (IE)**
• **Rothenbucher, Sandra**
  **80804 Munich (DE)**
• **Whyte, William**
  **Glen of Aherlow, Co. Tipperary E34 H248 (IE)**
• **Varela, Claudia**
  **Mission, TX 78574 (US)**
• **Robinson, Scott**
  **Galway, H91 V32F (IE)**
• **Mendez, Keegan**
  **Cambridge, MA 02139 (US)**

(74) Representative: **Purdylucey Intellectual Property
6-7 Harcourt Terrace
D02 FH73 Dublin 2 (IE)**

(54) **AN IMPLANTABLE MEDICAL DEVICE**

(57)    An implantable medical device having a soft tissue interfacing surface comprises at least one actuatable capsule having a soft tissue interfacing deflectable membrane configured for cyclical deflection upon actuation of the capsule to modulate the biomechanics of the soft tissue interface during use. The actuatable capsule may comprise an actuation chamber containing a first fluid, a therapeutic chamber containing a second fluid, a deflectable membrane separating the actuation chamber and therapeutic chamber, and an actuation conduit in fluidic communication with the actuation chamber for pneumatic actuation of the actuation chamber. The therapeutic chamber comprises the soft tissue interfacing deflectable membrane which is configured to cyclically deflect during actuation of the capsule and modulate the biomechanics of the soft tissue interface by altering one or more of strain, fluid flow and cellular activity in peri-implant tissue at the soft tissue interface. Methods of reducing fibrotic encapsulation of an implantable medical device are also described.

FIGURE 1C

EP 3 769 719 A1

## Description

### Field of the Invention

The present invention relates to an implantable medical device, in particular an implantable medical device modified to reduce fibrotic encapsulation. Also contemplated are methods of using the device, including methods of reducing fibrotic encapsulation of implantable medical devices

### Background to the Invention

The long-term performance of implantable medical devices is drastically limited by complex and unpredictable foreign body responses (FBR). Medical devices that depend on an interface with native tissue are particularly vulnerable, such as neural probes [1, 2], indwelling catheters [3], mammary implants [4], pacemakers [5], glucose biosensors [6-8] and drug and cell delivery devices [6, 9-12]. Such devices are often subject to a collection of biological host responses such as fibrosis, bacterial biofilm formation and inflammation, which can impair functionality. At present, device failure is expected and inevitable, and the costs, inconvenience and morbidity that it can impose on patients are largely accepted. The implantable medical devices market is mature and growing (compound annual growth rate of 8%), with an estimated worth of approximately 100 billion US dollars in 2019. Implantable medical devices have various failure rates that can be attributed to fibrosis, and can be as high as 30-50% for implantable pacemakers and 30% for mammoplasty prosthetics.

Once a device is implanted a complex series of events is initiated to protect the host from the 'foreign body'. Shortly after implantation, fibrinogen and other proteins bind to the surface of the device/foreign body, macrophages then bind and, over time, become multinucleated giant cells releasing inflammatory cytokines. In response to these signals, quiescent fibroblasts are transformed into myofibroblasts, which synthesize procollagen. The procollagen becomes crosslinked and this mature crosslinked collagen and other extracellular matrix proteins contribute to the formation of a dense fibrous capsule that becomes impermeable or hypo-permeable to many compounds. Various strategies have been investigated to mitigate the FBR, such as generating scaffolds that release small molecules, surface chemistry modifications, and modifying implant size and geometry. However, these treatments have met with limited success because of difficulty in incorporating these modifications in a range of implantable devices.

It is an object of the invention to overcome at least one of the above-referenced problems.

### Summary of the Invention

The Applicant has addressed the problem of fibrotic encapsulation of implanted medical devices by providing a soft tissue interfacing surface of the medical device with one or more and preferably an array of soft robotic capsules that can be actuated to cause cyclical deflection of a soft tissue interfacing membrane of the or each capsule that actively modulates the biomechanics of the biotic-abiotic interface by altering strain, fluid flow and cellular activity in the peri-implant tissue. The Applicant has shown that cyclical actuation of soft robotic capsules on a medical device implanted in a preclinical rodent model resulted in a significant reduction in fibrous capsule thickness (p=0.0362) compared to non-actuated controls, while the collagen density and orientation were not changed (p>0.05). A significant reduction in myofibroblasts (p=0.0036) in the actuated group was also demonstrated. Computational models quantified the effect of actuation on the reservoir and surrounding fluid. In a further aspect, adding a porous membrane (or a membrane that becomes transiently porous only upon actuation/deflection, or renders the native foreign body response porous) and a therapy reservoir to device of the invention, resulted in increased transport of a drug analog and enhanced pharmacokinetics and time to functional effect of an inotropic agent.

In a first aspect, the invention provides an implantable medical device having a soft tissue interfacing surface comprising at least one, and preferably a plurality of, actuatable capsule(s). The or each capsule comprises a soft tissue interfacing deflectable membrane configured for deflection when the capsule is actuated to modulate the biomechanics of the soft tissue interface, typically by altering one or more of displacement, force, strain, fluid flow and cellular activity in peri-implant tissue at the soft tissue interface.

The actuatable capsule may be configured for mechanical or electrical actuation, for example by inflation (i.e. cyclical inflation) to cause deflection of the soft tissue interfacing deflectable membrane. Alternative methods of mechanical actuation of the capsule are envisaged. For example, ultrasound may be employed to cause deflection of the soft tissue interfacing deflectable membrane. The capsule may include an ultrasound emitter configured to generate a shock wave in the fluid in the capsule by cavitation, leading to deflection of the deflectable membrane. Alternatively, an external ultrasound emitter may be employed to actuate the capsule. In another embodiment, part of the capsule (for example the soft tissue interfacing deflectable membrane) may comprise an actuating material (for example a charged material) that is configured to actuate the capsule when electrically stimulated. The actuating material may be a piezo-electric material. The deflectable membrane may comprise actuating material.

The actuatable capsule typically comprises:

an actuation chamber containing a first fluid;
a therapeutic chamber containing a second fluid that optionally may be replenishable from an external reservoir or transcutaneously;
a deflectable membrane separating the actuation chamber and therapeutic chamber; and
actuation means in fluidic communication with the actuation chamber for actuation of the actuation chamber,

wherein the therapeutic chamber comprises the soft tissue interfacing deflectable membrane.

In one embodiment, the actuation means comprises an actuation conduit in fluidic communication with the actuation chamber for fluidic actuation of the actuation chamber,

In one embodiment, the actuation chamber is defined by a non-deflectable membrane and the deflectable separating membrane.

In one embodiment, the therapeutic chamber is defined by the deflectable separating membrane and the soft tissue interfacing deflectable membrane.

In one embodiment, the capsule is a millisized capsule. However, it will be appreciated that larger or small capsules may be employed, for example micron-sized capsules or centimetre-sized capsules.

In one embodiment, the soft tissue interfacing surface comprises a plurality of capsules, for example at least 5, 10, 15, 20, 25, 30, or 40. Preferably, the plurality of capsules is arranged in an array.

In one embodiment, the soft tissue interfacing surface of the medical device comprises a skin that can optionally be retro-fitted to the implantable medical device. The skin can cover all or part of a tissue interfacing surface of the implantable medical device. The skin can be pre-shaped to conform closely to the soft tissue interface. For example, the skin can be shaped to conform to a surface of a tissue implant, for example a mammary implant. The skin can also be provided in the form of a sheath, i.e. configured to conform to a conduit implant (for example an indwelling catheter, pacemaker leads, or a neural implant). It will be appreciated that the skin can be configured to cover all or only a part of the medical device, and can be attached to the medical device during manufacture or retro-fitted to the implant prior to use.

In one embodiment, the or each capsule is integrally formed on a surface of the medical device.

In one embodiment, the soft tissue interfacing deflectable membrane is disposed on the soft tissue interfacing surface with the soft tissue interfacing deflectable membrane substantially flush with the soft tissue interfacing surface.

In one embodiment, the or each capsule is dome shaped having a substantially flat base provided by the soft tissue interfacing deflectable membrane. It will be appreciated that the capsule may have other shapes, for example cylindrical or rectangular or any other shape.

In one embodiment, the or each capsule comprises a dome shaped actuation chamber and a smaller dome shaped therapeutic chamber disposed within the actuation chamber.

The deflectable membrane is generally non-porous. However, in some embodiments, it may be porous (i.e. porous to active agent contained within the second fluid), or may be actuated to become porous (for example stretched). In one embodiment, the deflectable membrane is configured to be non-porous in a resting stage, and become porous when deflected. Thus, in one embodiment, the or each capsule comprises a therapeutic conduit in fluidic communication with the capsule (in one embodiment in fluidic communication with the therapeutic chamber) for delivery of an active agent (the active agent may be therapeutic or diagnostic), and in which the soft tissue interfacing deflectable membrane is typically porous or configured to become porous when it is actuated/deflected. This allows drug in the capsule/therapeutic chamber to be delivered by diffusion or by pumping. The use of a deflectable membrane that becomes porous only during actuation/deflection, allows for intermittent drug release.

In one embodiment, the or each capsule comprises a second actuation conduit in fluidic communication with the actuation chamber. This allows the capsule to be connected in series to a pump, with fluid being pumped into the actuation chamber through one conduit and exiting the chamber through the second conduit.

In one embodiment, the device comprises an implantable pump operatively connected to the first and/or second actuation conduit. While the use of an implantable pump is preferred, it will nonetheless be appreciated that a remote, non-implantable pump may be employed to fluidically actuate the actuation chamber and cause cyclical deflection of the soft tissue interfacing membrane. In one embodiment, the pump is a piezoelectric pump.

When a plurality of capsules is employed, the pump may be operatively connected to a plurality of the capsules. The pump may be connected to the plurality capsules in series or in parallel.

In one embodiment, the pump is operatively connected to the first and second conduits in series and is configured to pump fluid to the first conduit and receive fluid from the other conduit.

In one embodiment, the second conduit comprises a fluidic resistor configured to resist fluid flow. This helps with actuation of the actuation chamber by helping maintain pressure in the actuation chamber when the pump is actuated.

In one embodiment, the second conduit comprises a fluidic capacitor typically disposed distally of the fluidic resistor (i.e an expandable chamber distal of the fluidic resistor that helps release pressure in the first fluid and allow deflation of the actuation chamber).

In one embodiment, the implantable medical device is selected from a tissue implant, an indwelling catheter, a pacemaker, a biosensor, a drug or cell delivery device, and a neural probe. The or each capsule may be integrally formed as part of a soft tissue interfacing surface of the implant, or may be provided as a "skin" that can be attached to all or part of the soft tissue interfacing surface of the medical device during manufacture, or retro-fitted after manufacture and prior to implantation.

In another aspect, the invention provides a retro-fit skin for forming an implantable medical device of the invention. The retro-fit skin is configured to adhere to and cover at least part of a surface of the implantable medical device and form a soft tissue interfacing surface on the medical device, and comprises at least one inflatable capsule. The capsule typically comprises:

an actuation chamber containing a first fluid;
a therapeutic chamber containing a second fluid that can optionally be replenished from an internal reservoir or transcutaneously;
a deflectable membrane separating the actuation chamber and therapeutic chamber; and actuation means in fluidic communication with the actuation chamber for actuation of the actuation chamber,
wherein the therapeutic chamber comprises a soft tissue interfacing deflectable membrane.

In one embodiment, the actuation means comprises an actuation conduit in fluidic communication with the actuation chamber for fluidic actuation of the actuation chamber,

In one embodiment, the skin is formed from a soft polymeric film, for example a silicone skin.

In one embodiment, the actuation chamber is defined by a non-deflectable membrane and the deflectable separating membrane.

In one embodiment, the therapeutic chamber is defined by the deflectable separating membrane and the soft tissue interfacing deflectable membrane.

In one embodiment, the capsule is a millisized capsule.

In one embodiment, the skin comprises an array of capsules.

The skin can cover all or part of a tissue interfacing surface of the implantable medical device. The skin can be pre-shaped to conform closely to the soft tissue interface. For example, the skin can be shaped to conform to a surface of a tissue implant, for example a mammary implant. The skin can be provided in the form of a sheath, i.e. configured to conform to a conduit implant (for example an indwelling catheter, pacemaker leads, or a neural implant). It will be appreciated that the skin can be configured to cover all or only a part of the medical device, and can be attached to the medical device during manufacture or retro-fitted to the implant prior to use.

In one embodiment, the or each capsule is integrally formed on a surface of the medical device.

In one embodiment, the soft tissue interfacing deflectable membrane is disposed on the skin with the soft tissue interfacing deflectable membrane substantially flush with the soft tissue interfacing surface of the skin.

In one embodiment, the or each capsule is dome shaped having a flat base provided by the soft tissue interfacing deflectable membrane. It will be appreciated that the capsule may have other shapes, for example cylindrical or rectangular or any other shape.

In one embodiment, the or each capsule comprises a dome shaped actuation chamber and a smaller dome shaped therapeutic chamber disposed within the actuation chamber.

In one embodiment, the or each capsule comprises a therapeutic conduit in fluidic communication with the therapeutic chamber for delivery of an active agent (the active agent may be therapeutic or diagnostic), and in which the soft tissue interfacing deflectable membrane is porous. In this manner, deflection of the porous membrane causes an increase in transport of the active agent into the surrounding tissue.

In one embodiment, the or each capsule comprises a second actuation conduit in fluidic communication with the actuation chamber. This allow the capsule to be connected in series to a pump, with fluid being pumped into the actuation chamber through one conduit and exiting the chamber through the second conduit.

In one embodiment, the device comprises an implantable pump operatively connected to the first and/or second actuation conduit. While the use of an implantable pump is preferred, it will nonetheless be appreciated that a remote, non-implantable pump may be employed to pneumatically actuate the actuation chamber and cause cyclical deflection of the soft tissue interfacing membrane.

In one embodiment, the pump is operatively connected to the first and second conduits in series and is configured to pump fluid to the first conduit and receive fluid from the other conduit.

In one embodiment, the second conduit comprises a fluidic resistor configured to resist fluid flow. This helps with actuation of the actuation chamber by helping maintain pressure in the actuation chamber when the pump is actuated.

In one embodiment, the second conduit comprises a fluidic capacitor (i.e an expandable chamber distal of the fluidic resistor that helps release pressure in the first fluid and allow deflation of the actuation chamber).

The invention also provides an implantable medical device comprising a retro-fit skin of the invention adhered to at least a part of the soft tissue facing surface of the medical device.

In another aspect, the invention provides a method (typically a method of reducing fibrotic encapsulation of an implantable medical device), comprising the steps of:

implanting an implantable medical device according to the invention into a subject with the soft tissue interfacing surface of the device in contact with soft tissue of the subject; and

cyclically actuating the or each actuatable capsule disposed on the soft tissue interfacing surface to effect cyclical deflection of the soft tissue interfacing membrane,

whereby the cyclical deflection of the soft tissue interfacing membrane modulates the biomechanics of the soft tissue interface by altering one or more of displacement, force, strain, fluid flow and cellular activity in peri-implant tissue at the soft tissue interface.

In one embodiment, the method comprises cyclically actuating the actuation chamber to effect cyclical deflection of the soft tissue interfacing membrane.

In one embodiment, the actuation chamber is actuated at an actuation pressure of 0.5 to 5.0 PSI, and preferably 0.5 to 3.0 PSI

In one embodiment, the actuation chamber is actuated at a frequency of 0.1 to 10.0 Hz, preferably 0.5 to 5 Hz, and more preferably 0.5 to 1.5 Hz.

In one embodiment, the cyclical actuation of the actuation chamber is performed continuously or periodically, for example once every hour, day, week or month.

In one embodiment, the or each capsule comprises a therapeutic conduit in fluidic communication with the therapeutic chamber for delivery of an active agent (the active agent may be therapeutic or diagnostic), and in which the soft tissue interfacing deflectable membrane is typically porous or configured to be transiently porous during actuation/deflection, wherein the method includes a step of delivering or pumping active agent into the therapeutic chamber to refill or replenish the chamber.

In one embodiment, the implantable medical device is a cell encapsulation device.

Other aspects and preferred embodiments of the invention are defined and described in the other claims set out below.

Brief Description of the Figures

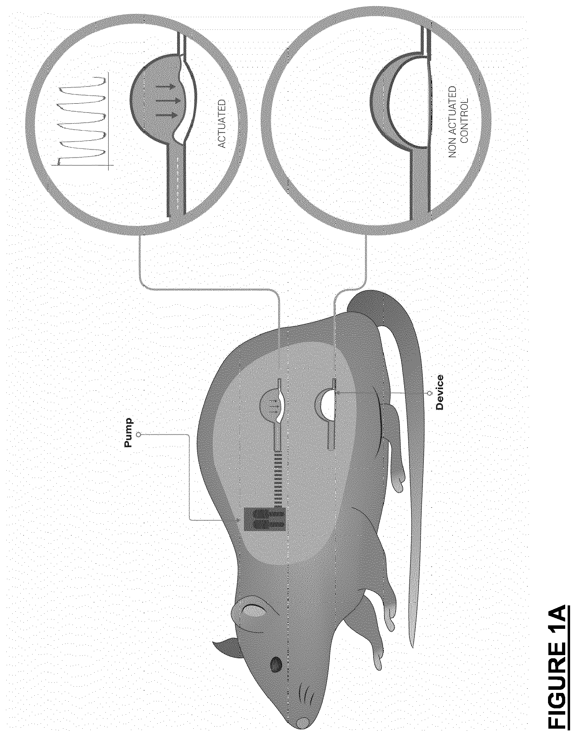

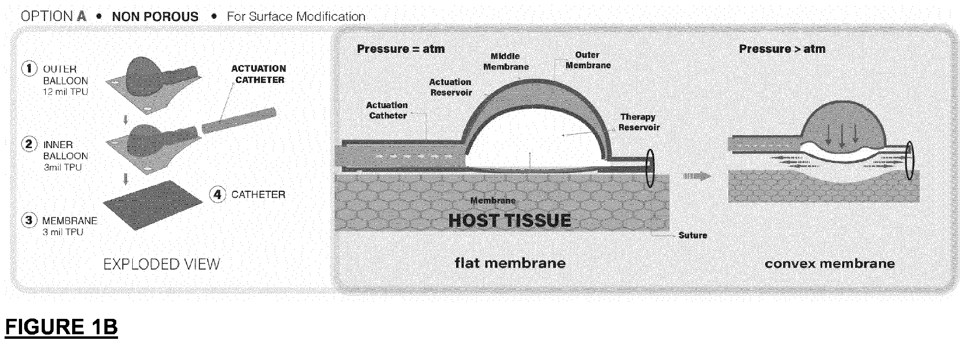

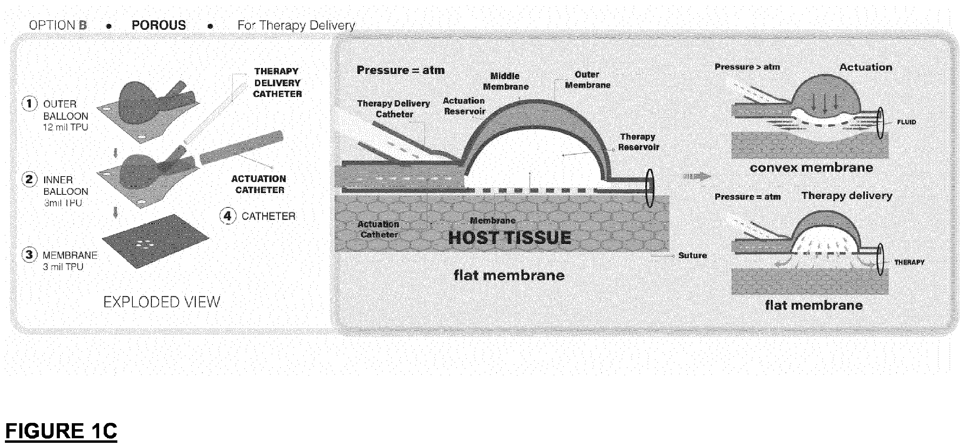

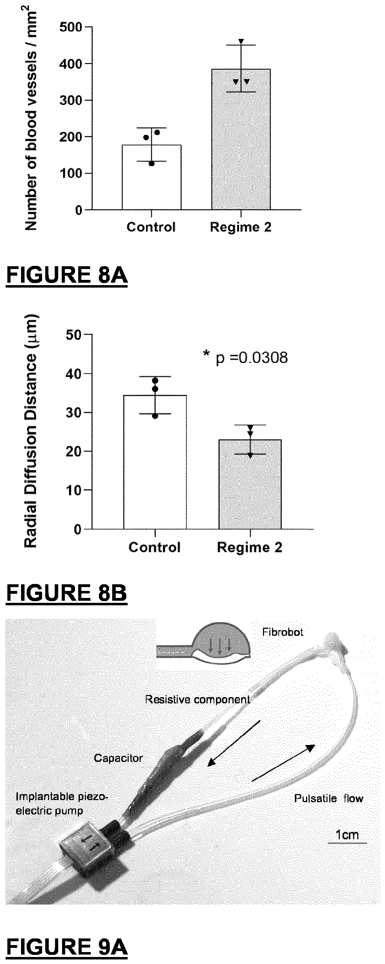

**Figure 1:** An overview of the device and the proposed mechanism of action. A) Implantable system showing a side-by-side implantation of a control and actuation group. B) Non-porous configuration for foreign body modulation for implantable devices. C) Porous configuration for therapy delivery. The device can be cyclically actuated by an implantable piezoelectric pump, and we demonstrate this in a rat model (Fig. 1a, Fig. 8). Cyclical pressurization results in a predictable, regime-specific deflection and strain of the lower functional membrane, see Fig. 1b and c. Multiple such reservoirs can be incorporated into a thin, conformable matrix that can be designed to be part of, or surround, implantable devices to reduce the host FBR. The proposed mechanism of action of the individual reservoirs is shown in Fig. 1b and c. When the actuation reservoir is pressurized through the actuation line, the volume, and then pressure of the chamber increases, causing a downward deflection of the middle, and subsequently the lower membrane. Actuation causes deflection and strain of the membrane, and results in fluid flow at the tissue interface (blue arrows Fib. 1b and c) which is proposed to interfere with cell activity and initiation of the fibrotic response. Two configurations of the device are described - one version with an impermeable, non-porous lower membrane (Fig. 1b), which would be incorporated in a sheath as a surface modification (suited toward implants with a structural role), and a second version with a permeable, porous membrane (Fig.1c) that incorporates an additional therapy delivery line to the lower reservoir to allow transport of therapy into the tissue (suited for drug delivery or biosensing applications).

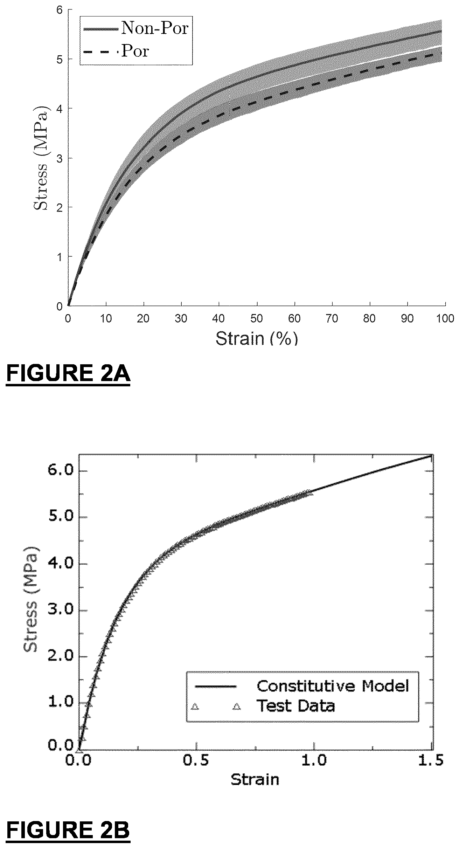

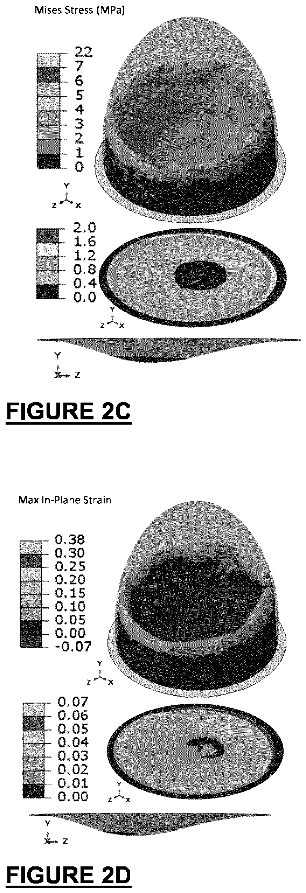

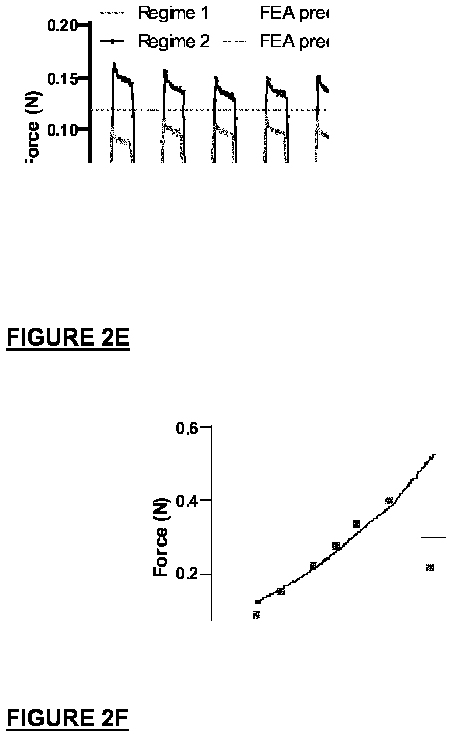

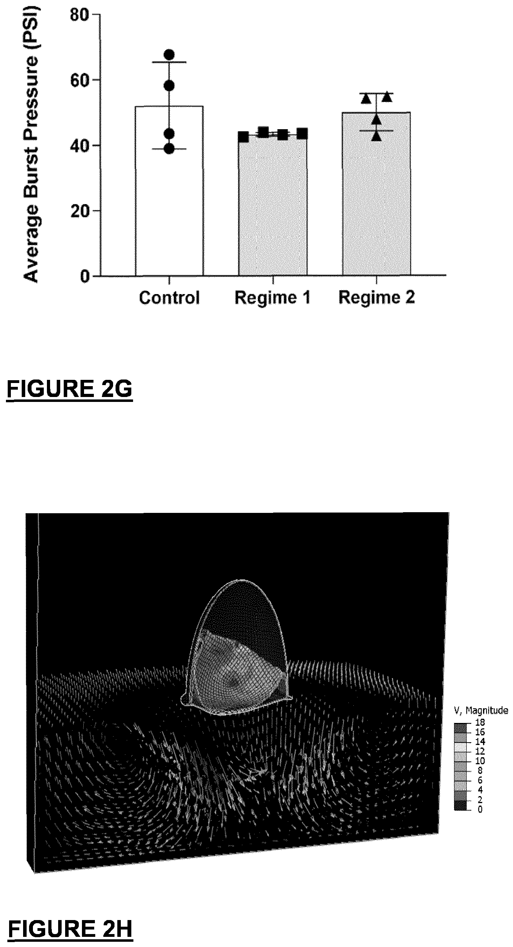

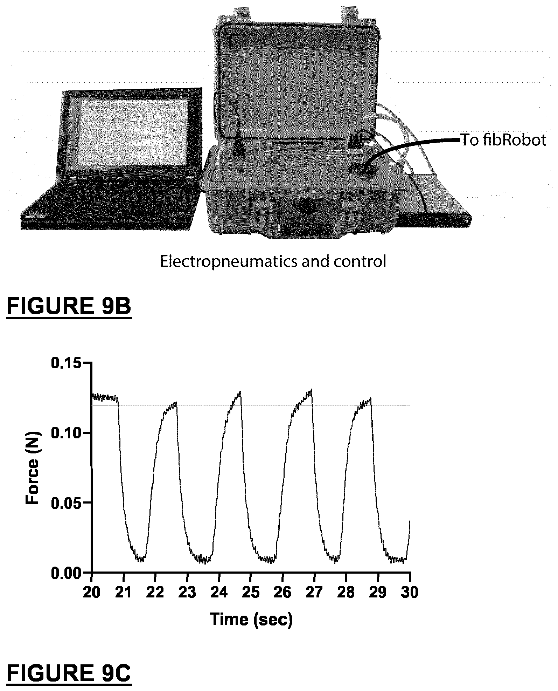

**Figure 2:** Computational and experimental characterization: A) Stress/strain plots for porous and non-porous thermoplastic urethane specimens. B) Fitting of the porous data to the Ogden hyperelastic model. C) Mises stress contour plots for the overall device, and the lower membrane (top-down and side view). D) Maximum in-plane strain for the overall device, and the lower membrane (top-down and side view). E) Experimental cyclical force measurements two actuation regimes (regime 1 = 1 PSI at 1 Hz, regime 2 = 2 PSI at 1 Hz), and finite element predicted force for each. F) Relationship between input pressure and actuation force as measured experimentally, and predicted with finite element analysis (FEA). G) Burst failure of device of the invention reservoir after actuating at regime 1 or regime 2 for 8400 cycles. n=3/group, data are mean $\pm$ standard deviation p>0.05. H) Smooth particle hydrodynamic model showing the direction and magnitude of fluid flow surrounding device of the invention during actuation. The thermoplastic urethane used for the membrane (porous and non-porous) was tensile tested according to ASTMD638. The resulting stress vs strain plots are shown in Fig. 2a. The data was fitted to a hyperelastic material model with the Ogden model being selected as the best fit (Fig. 2b, Fig. 9). With this material model as an input, a finite element analysis model was created to demonstrate the overall strain (Fig. 2c), and resulting stress (Fig.2d) in the inner and outer balloons and membrane. This was repeated for the non-porous and porous membranes with maximum in-plane strain of 6.4% and 8.6% respectively (Fig. 9). To validate the computational models, we measured the force that the membrane applies to interfacing tissue, using a custom acrylic holder (Fig. 9) to consistently support the device in a materials tester, with the functional membrane in contact with the upper load cell. The cyclical forces for two regimes (regime 1 = 1 PSI at 1 Hz, regime 2 = 2 PSI at 1 Hz) were recorded, and peak forces were compared to each other, and their corresponding finite element models, showing an excellent match between measured and predicted values (Fig. 2e). To further characterize the possible range of input pressures, higher pressures and corresponding forces were also modelled computationally and experimentally (Fig. 2f). When we compared the burst pressure of the devices after they were subjected to the in vivo actuation regime (8400 cycles) normality of distribution was observed. A one way analysis of variance (ANOVA) was performed with post hoc Tukey's test yielding no statistical difference between groups (p=0.3368) (Fig. 2g) indicating that the applied actuation regimes fall within the fatigue limit of the devices. Finally, we used a smooth particle hydrodynamics model to quantify the fluid perturbations from device actuations during device actuation in fluid (Fig. 2h).

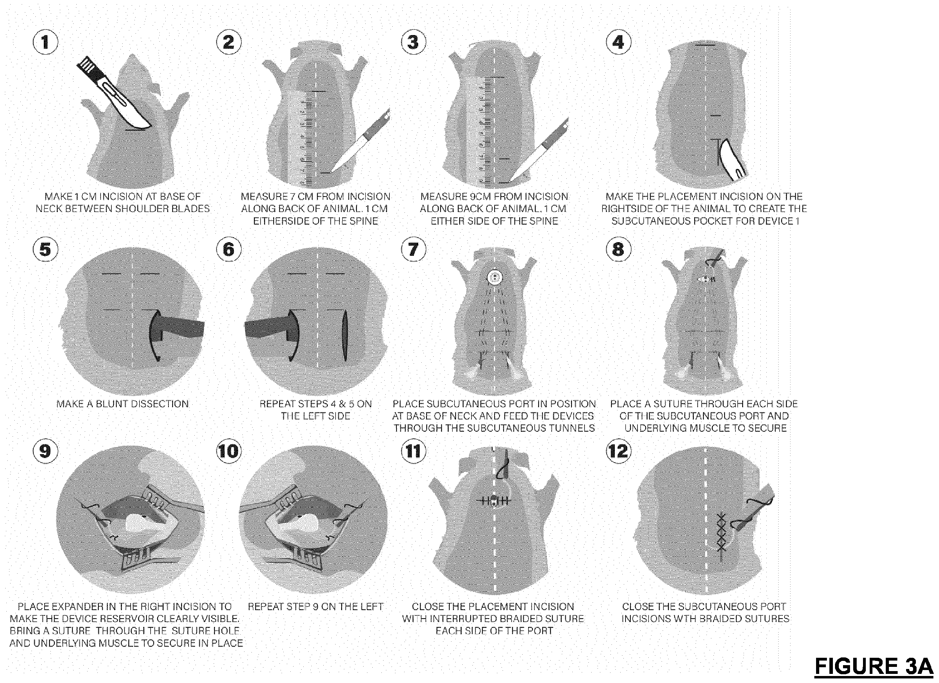

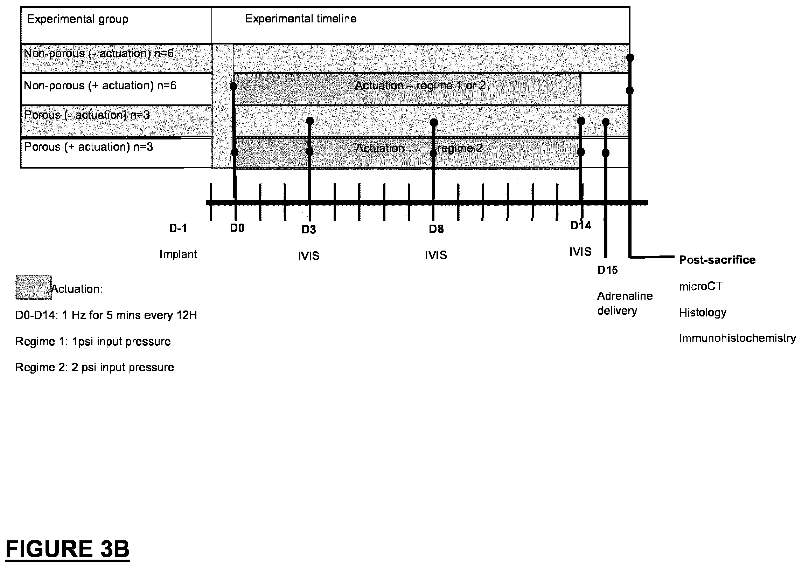

**Figure 3:** Pre-clinical implementation of device of the invention. A) Device implantation procedure. Each animal has two implanted reservoirs subjected to no actuation, regime 1 or regime 2. B) Timeline for in vivo studies for porous and non-porous device of the inventions.

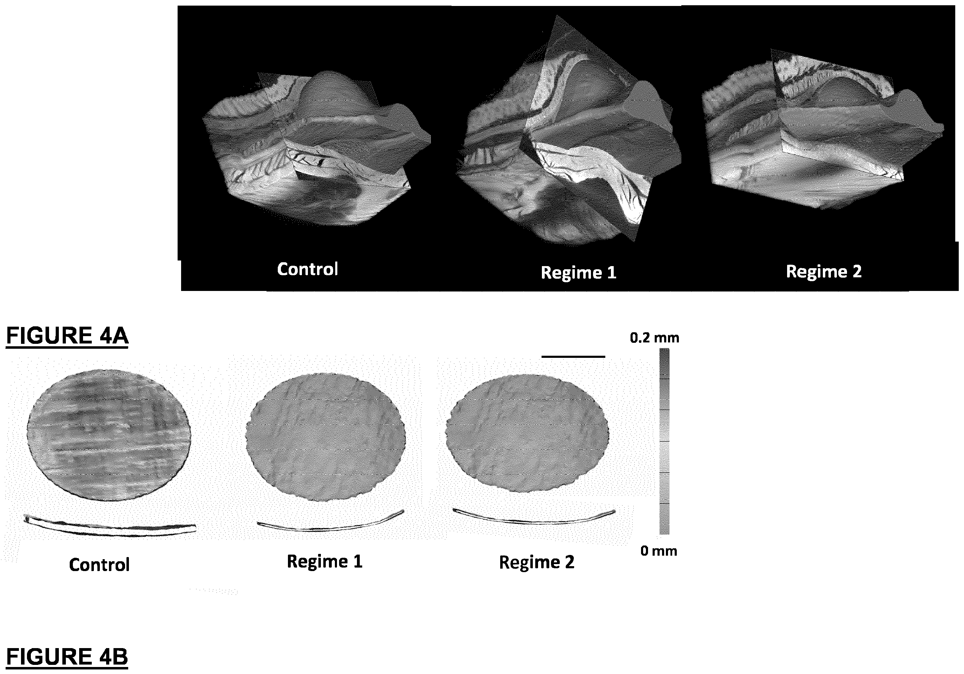

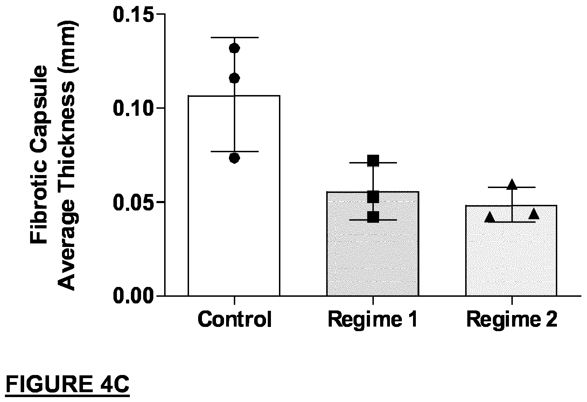

**Figure 4:** device of the invention reduces the fibrous capsule thickness in vivo. A) MIMICs software (Materialise, Leuven) reconstruction of soft tissue stained with PMA and imaged with microCT, where the device is shown in blue and the fibrotic capsule is shown in purple. B) Thickness analysis in MIMICs where surface shell elements are shown, and thickness is calculated as the distance between them. C) Average thickness across the fibrotic capsule, n=3/group, data are mean $\pm$ SD, *p<0.05. On day 15 the devices were explanted en bloc with surrounding tissue and stained with phosphomolybdic acid (PMA) to enable visualization of collagen dense tissues. Images of the device and surrounding soft tissue were acquired using microCT. MIMICs (Materialise) software was used for segmentation of the device space (Fig. 4a shown in blue) and the fibrotic capsule present on the membrane of the device (Fig. 4a shown in purple). Volumetric reconstructions of the fibrotic capsule were generated, and a thickness analysis was performed to identify mean capsule thickness. Normal distribution was observed in all groups and a one-way ANOVA was carried out with post hoc Tukey's analysis. A downward trend in the mean capsule thickness was seen when the devices were actuated compared to the non-actuated control. A significant reduction in the mean fibrotic capsule thickness was seen when the device was actuated to regime 2 compared to the control (0.0486 $\pm$ 0.009 mm vs. 0.106 $\pm$ 0.032 mm, *p = 0.0362), see Fig. 4b and c.

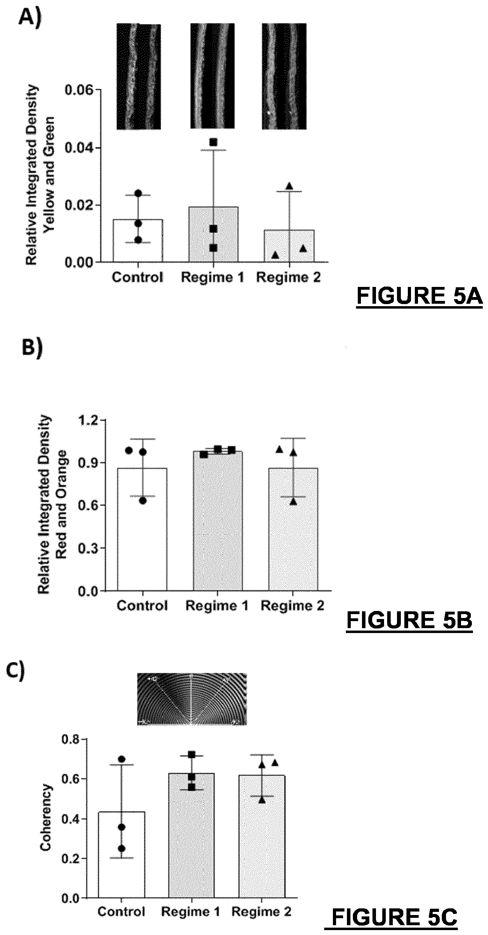

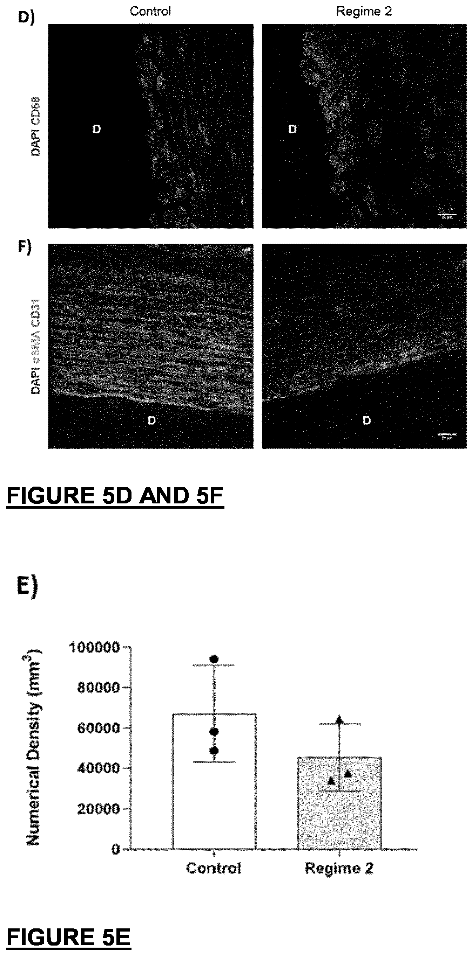

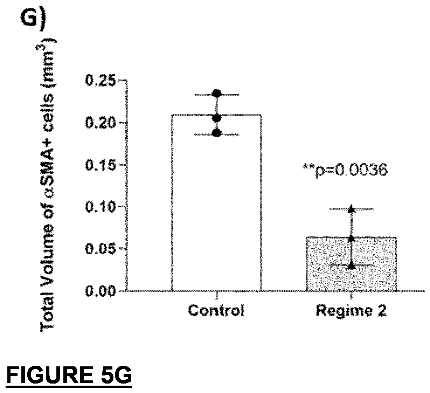

**Figure 5:** Histological analysis of the fibrous capsule. A) Relative integrated density of yellow and green fibres (signifying immature collagen) from polarized light microscopy images of the fibrous capsule in response to different treatments, with representative images shown. B) Relative integrated density of red and orange fibres (signifying mature collagen) from polarized light microscopy images of the fibrous capsule. C) Coherency of fibrous capsule based on polarized light microscopy. D) Representative immunofluorescent images of capsular tissue sections stained with CD68 (Red = CD68, Blue = Hoechst). E) Numerical density of CD68 stained macrophages in different treatment groups. F) Representative immunofluorescent images of capsular tissue sections stained with $\alpha$SMA (Blue = DAPI, Green = $\alpha$SMA, Red = CD31). G) Total volume of $\alpha$SMA+ cells (mm$^3$). n=3/group, Data are mean $\pm$ SD, **p<0.01

To characterize the effect of actuation on the cellular constituents involved in the FBR, devices explanted on day 15 were sectioned for histological analysis. Collagen fibres are characteristically birefringent which is enhanced with picrosirius staining. Polarized light microscopy was used to assess the quality and organisation of the resulting fibrous capsule after staining. Quantification of birefringent fibres, using color threshold segmentation for mature fibres (red/orange) and immature fibres (green), showed normal distribution and a one-way ANOVA with post hoc Tukey's test yielded no statistically significant difference between any of the groups (p > 0.05), irrespective of polarization color (Fig. 5a, b). Quantification of the directional uniformity (coherency) of the collagen fibres showed normal distribution and a one-way ANOVA with post hoc Tukey's test yielded no statistically significant difference in the fraction of fibres (p=0.3053) (Fig 5c), suggesting actuation did not result in a compositional change in the collagen within the fibrous capsule.

Tissue sections were stained with CD68, a pan-macrophage marker, to assess impact of actuation on the macrophage response at the tissue-device interface. Immunofluorescent images (Fig. 5d) were acquired for the control and actuated (regime 2) devices and a stereology approach was utilized to provide a quantitative analysis of macrophage number. Numerical density (Fig. 5e) of macrophages was calculated. Normal distribution was observed and an unpaired t-test was carried out with no statistically significant difference observed between groups (p=0.2703), suggesting that actuation does not influence macrophage number at the device surface.

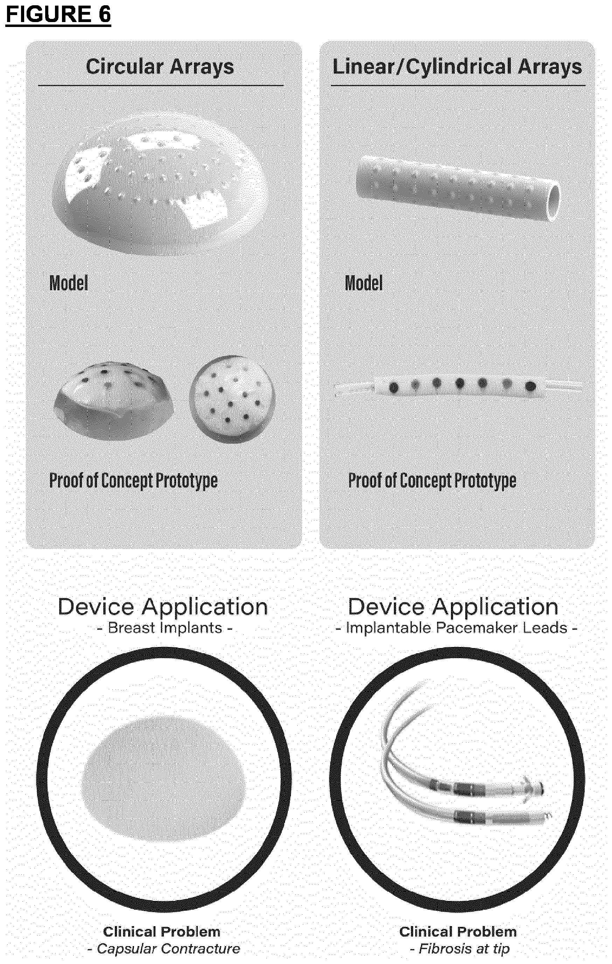

**Figure 6:** Arrays of non-porous device of the invention reservoirs to reduce complications associated with implantable devices. Circular arrays are encapsulated around a breast implant, and a linear array is integrated into a cylindrical structure, ultimately envisioned for pacemaker leads. Device of the invention arrays are shown in colour, with each colour representing an individual actuation line. Incorporating arrays of non-porous device of the invention reservoirs into medical devices that suffer from an adverse host response helps to reduce these effects. We demonstrate physical embodiments of circular and linear arrays of device of the invention, with reservoirs in series and in parallel, by encapsulating multiple reservoirs (manufactured from thermoplastic urethane or silicone - shown in colour in Fig. 6), and their actuation lines in a silicone sheet around a breast implant, and tubular structure that is ultimately envisioned for pacemaker leads or indwelling catheter lines (Fig. 6).

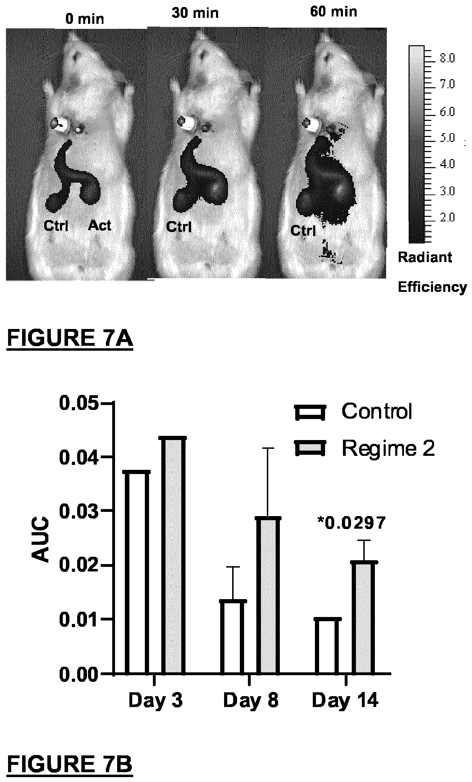

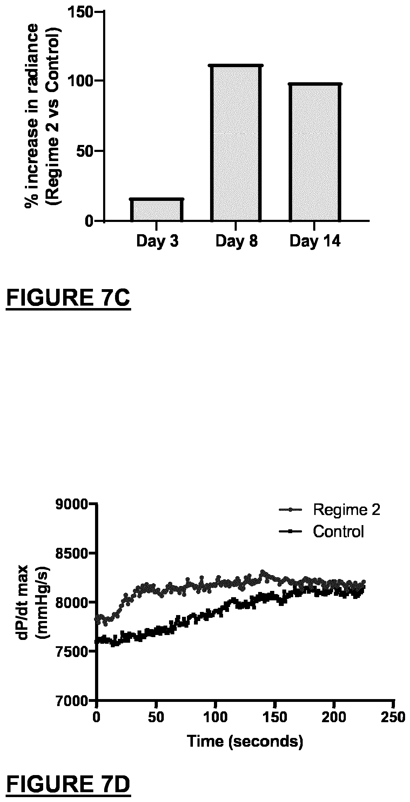

**Figure 7:** Enhanced pharmacokinetics through porous device of the invention with actuation. A) Images from the In Vivo Imaging System (IVIS) at day 14 at 0 minutes, 30 minutes and 60 minutes showing the implanted actuation (regime 2) and control groups. B) Area under the curve at day 3, 8 and 14 for actuated and control groups n=1-4/group/timepoint, Data are mean $\pm$ SD, *p<0.05. C) Percentage increase of actuation group over control at each timepoint n=1-4/group. D) dP/dt max, a measure of ventricle contractility for the actuated (Regime 2) and control device of the intervention reservoirs in a representative animal. Ctrl=control, Act=act, AUC = area under curve. To investigate the transport of therapy through the resulting fibrous capsule, a therapy analogue Genhance (Perkin Elmer) was injected though the therapy delivery catheter of a control and actuated (regime 2) porous device and subsequently through the porous membrane to the surrounding tissue at day 3, 8 and 14. Fluorescent in vivo imaging was conducted for 1 hour after delivery of drug analogue (Fig. 7a). Fluorescence (radiant efficiency) over time was plotted for each animal, for the control and actuated (regime 2) devices (Fig. 7b). A 2-way ANOVA with Tukey's post-hoc multiple comparison test of the area under the curve revealed that there was a significant decrease in the amount of drug analogue delivered to an area of tissue surrounding the implant from day 3 to 14 (*p=0.0297), as expected due to the impedance of drug diffusion due to the FBR (Fig. 7b). However, this reduction was attenuated with actuation, as diffusion was not impeded to the same extent as in the actuated (regime 2) devices as they showed higher delivery of drug analogue compared to the control at each time-point (Fig. 7b,c).

**Figure 8:** Implantable actuation system: A) Schematic of the piezo-electric pump and the closed loop cycle to device of the invention, a resistive component, and a capacitor or compliant component. B) Test system with external electropneumatic control box. C) Measured pulsatile force generation from implantable pump compared to measured average peak force with external control box.

**Figure 9:** Test set-up for force characterization and porous vs non-porous computational models. A) Acrylic holder for supporting reservoir and ensuring that lower functional membrane is in contact with upper cross-head. B) Test set-up in mechanical tester. C) Strain energy density function for Ogden model. D) Light microscopy of laser-cut porous membrane. Pore diameters were 10 micron with spacing of 200 microns. E) Boundary conditions applied to the finite element model. F) In-plane strain and G) Mises stress for the porous vs non-porous device of the inventions showing relatively small differences.

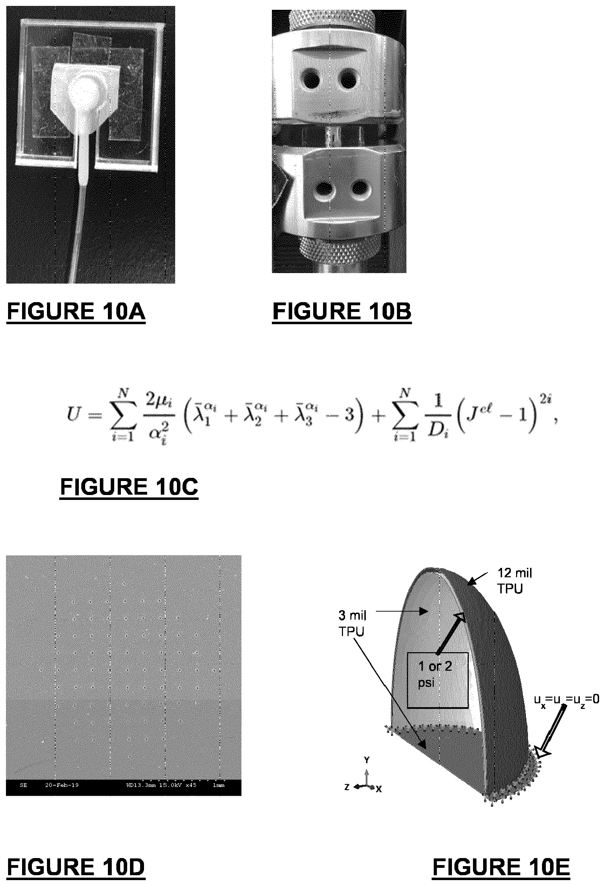

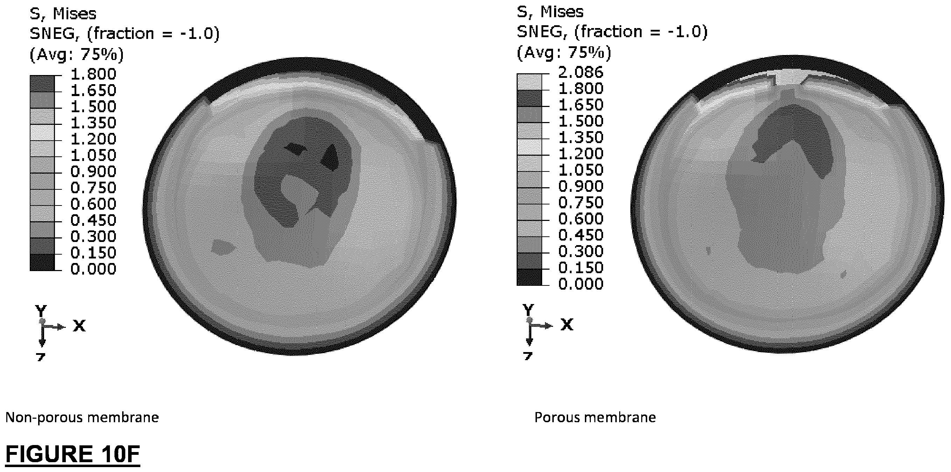

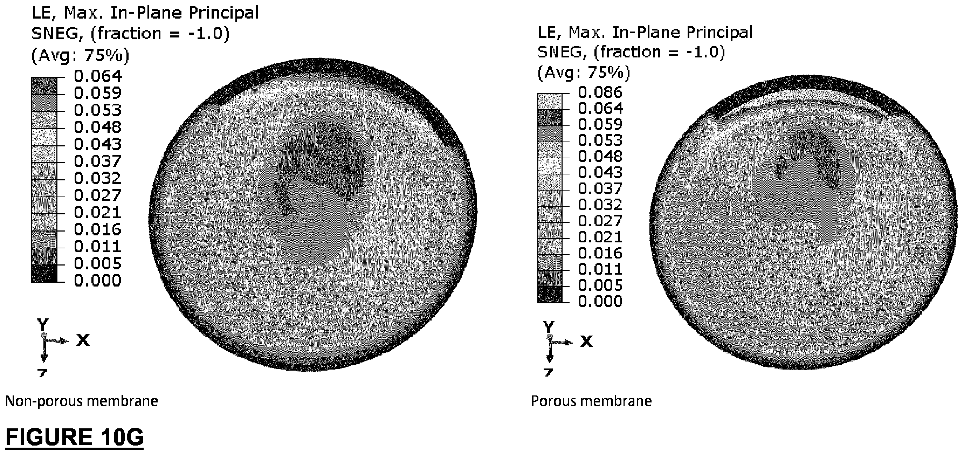

**Figure 10:** Actuation caused an increase in blood vessels. A) Number of blood vessels/mm2 B) Radial diffusion distance for regime 2 compared to control. n=3/group, data are mean $\pm$ standard deviation, *p<0.05

Neo-vascularization was also assessed in the fibrous capsule by staining for CD31 and counting the number of vessels present around the control and actuated (regime 2) devices. For number of blood vessels per unit area data, normal distribution was observed in control but not in regime 2. Man Whitney U test was performed yielding no statistical significance (p=0.100). However, there was an increasing trend in the number of vessels in the regime 2 devices compared to the control. For radial diffusion distance, normal distribution was observed in both control and regime 2. An unpaired t-test was performed and a significant decrease in the radial diffusion distance in the regime 2 devices compared to the control group (*p=0.0308) indicating an increased vascularity of the capsule following actuation (Fig. S3b).

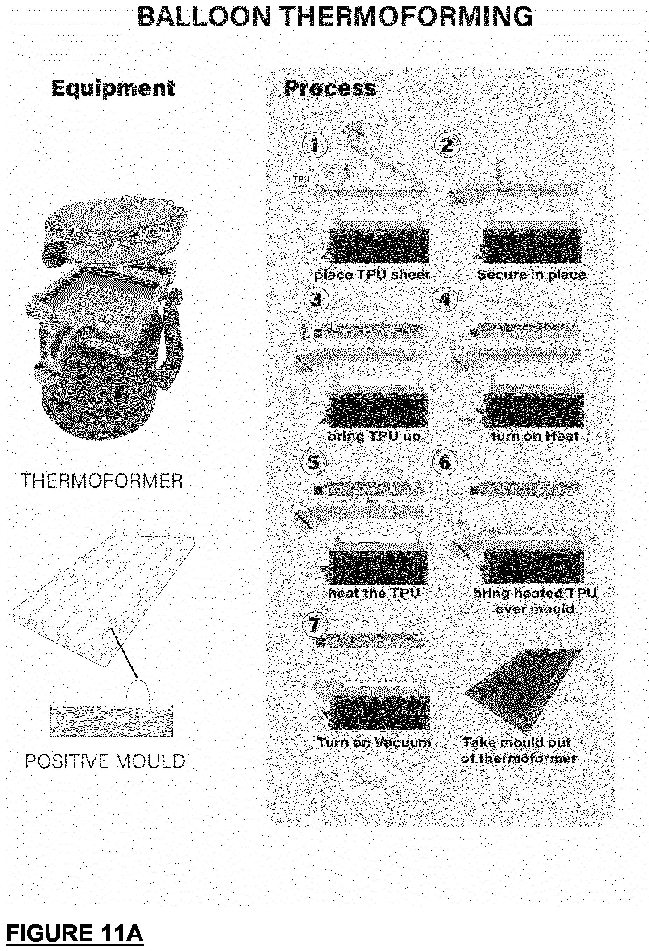

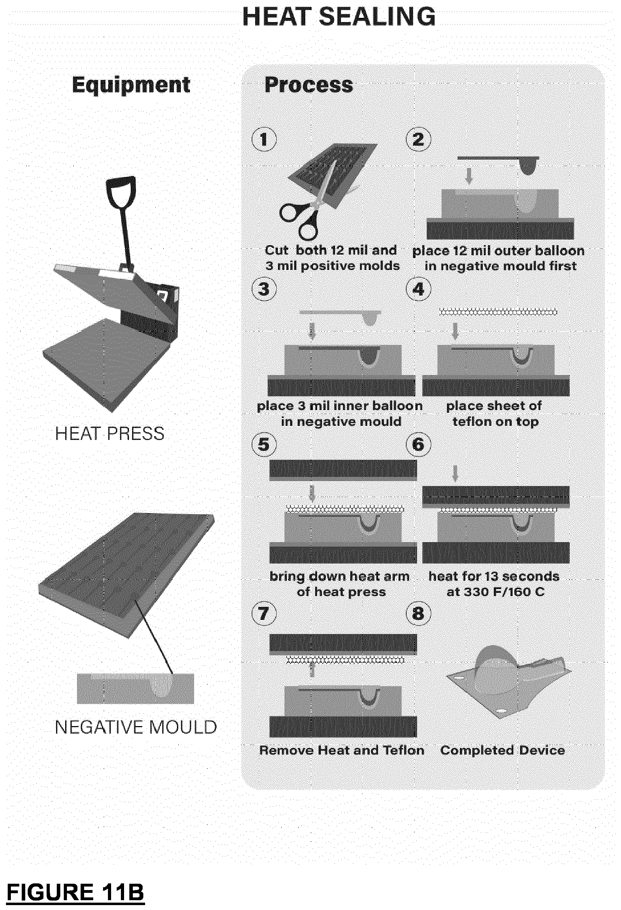

**Figure 11:** Device manufacture procedure which involves two step - balloon thermoforming and heat sealing. Balloon Thermoforming: 1) Thermoplastic urethane (TPU) sheets (3 mil and 12 mil) were mounted separately in a thermal former with a vacuum platform and a positive 3D printed mould was placed on the platform, 2) The TPU sheet was secured in place, 3) The TPU was brought close to the heating element, 4) the heat was turned on, 5) the TPU was heated until it sags in the centre, 6) the platform was lowered over the positive mould, 6) the vacuum was applied to form the sheet over the positive mould, 8) the formed TPU was removed from the positive mould. Heat sealing: 1) The formed TPU was cut out, 2) 12 mil TPU was placed in a 3D printed negative mould, 3) 3 mil TPU was placed in a 3D printed negative mould on top of the 12 mil TPU, 4) A teflon strip was inserted into the groove of the negative mould between relevant layers of TPU to keep the catheter channels open during the following heat-sealing 5) a 3 mil TPU membrane (porous or non-porous) was placed on top and a layer of Teflon was played on top to protect the assembly, 5) the arm of the heat press was brought down on top of the mould, 6) the assembly was heat sealed with a heat transfer machine, 7) the heat arm and Teflon was removed, 8) device reservoir was complete. Catheter tubing was inserted into relevant channels and heat shrink tubing was used to seal the devices.

Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa*. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, age, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure. Improvement may be observed in biological / molecular markers, clinical or observational improvements. In a preferred embodiment, the methods of the invention are applicable to humans, large racing animals (horses, camels, dogs), and domestic companion animals (cats and dogs).

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, camels, bison, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human. As used herein, the term "equine" refers to mammals of the family *Equidae,* which includes horses, donkeys, asses, kiang and zebra.

"Implantable medical device" should be understood to mean an indwelling medical device that depends on an interface with native tissue. Examples include neural probes [1, 2], indwelling catheters [3], mammary implants [4], pacemakers [5], glucose biosensors [6-8] and drug and cell delivery devices [6, 9-12]. Such devices are often subject to a collection of biological host responses such as fibrosis, bacterial biofilm formation and inflammation, which can impair functionality. At present, device failure is expected and inevitable, and the costs, inconvenience and morbidity that it can impose on patients are largely accepted. The present invention proposes modifying the implantable medical device to reduce FBR by incorporating one or more capsules on a soft tissue interfacing surface of the device. The capsules include a soft tissue interfacing deflectable membrane configured for deflection (typically cyclical deflection) when the capsule is actuated, which alters fluid flow and strain in the soft tissue interface reducing FBR.

"Soft tissue interfacing surface" refers to a part of the surface of the implantable medical device that interfaces with soft tissue in a subject. This surface may form all or part of the surface of the medical device. It will be appreciated that the invention may also be employed in devices that interface with hard tissue, such as bone.

"Actuatable capsule" refers to a soft capsule having a soft tissue interfacing membrane configured for deflection when the capsule is actuated. Actuation of the capsule may involve inflation of the capsule, for example fluidic (pneumatic or hydraulic) inflation. Alternative methods of mechanical actuation of the capsule are envisaged. For example, ultrasound may be employed to cause deflection of the soft tissue interfacing deflectable membrane. The capsule may include an ultrasound emitted configured to generate shock waved in the fluid in the capsule by cavitation, leading to deflection of the deflectable membrane. Alternatively, an external ultrasound emitter may be employed to actuate the capsule. In another embodiment, the part of the capsule (for example the soft tissue interfacing deflectable membrane) may comprise an actuating material (for example a charged material) that is configured to actuate the capsule when electrically stimulated. The actuating material may be a piezoelectric material. The deflectable membrane may comprise actuating material. The capsule may be dome shaped, with the soft tissue interfacing membrane typically forming a base of the capsule. The capsule may comprise two chambers, an actuation chamber and a therapeutic chamber, separated by a deflecting separating membrane or barrier. The actuation chamber is generally disposed in the capsule adjacent the medical device, and the therapeutic chamber is generally disposed adjacent tissue. The actuation chamber is generally larger than the therapeutic chamber, with the therapeutic chamber disposed within the actuation chamber. The capsule is generally formed by a soft polymeric material, for example any thermoplastic or mouldable material such as a polyether film (i.e. TPU polyether). In one embodiment, the soft tissue interfacing membrane is porous to allow the second fluid in the therapeutic chamber pass through the membrane into the surrounding tissue. The size of the pores is variable and depends on the size of the active agent being delivered, and in one embodiment is sized between 5-15 microns.

"Millisized" as applied to an actuatable capsule refers to a capsule having a maximum diameter and height in the mm range, for example of not greater than 30mm (i.e. 1-30 mm), for example a diameter and height of about 5-20 mm or preferably about 10-15 mm. It will be appreciated that other sizes of capsule may be employed, for example capsules having a height and width in the micro range or in the cm range.

"Fluid" refers to a gas or a liquid, typically a gas which is preferably air or saline. The first and second fluids may be the same or different. Generally, the therapeutic chamber contains a gas.

"Array" refers to an arrangement of a plurality of capsules on the soft tissue interfacing surface of the implantable medical device. The array may be an ordered array. In one embodiment, the array is configured such that adjacent capsules are separated by a distance of 1-5 cm.

"Skin" or "retro-fit skin" refers to a cover or sheath member comprising one or more capsules and configured for adhering to all or part of a surface of the implantable medical device. The skin can be attached to the medical device during manufacture, or retro-fitted to the device after manufacture and prior to use. The skin can be provided separately to the medical device. The skin can be shaped to conform to a surface of the medical device, for example it can be shaped to conform to a breast implant, or may be cylindrical or a sheath to conform to an indwelling catheter. Generally, the soft tissue interfacing membrane of the capsules are flush with surface of the skin. The skin is generally soft and pliable, and may be formed from a soft polymer such as silicone.

"Active agent" refers generally to an agent or component that has a pharmaceutical or biological effect in a mammal, or diagnostically active agent such as an imaging dye or radioligand or antibody. Examples include glucose, insulin, drugs, for example, a drug to relieve pain such as non-steroidal anti-inflammatory drug (such as Ibuprofen, Ketoprofen or Naproxen), aspirin, acetaminophen, codeine, hydrocodone, an anti-inflammatory agent such as a steroidal anti-inflammatory agent, an anti-depressant, an anti-histamine, or an analgesic, polysaccharides, proteins, peptides, polypeptides, antigens, antibody (monoclonal or polyclonal), antibody fragments (for example an Fc region, a Fab region, a single domain antibody such as a nanobody or VHV fragment), a conjugate of an antibody (or antibody fragment) and a binding partner such as a protein or peptide, a nucleic acid (including genes, gene constructs, DNA sequence, RNA sequence, miRNA, shRNA, siRNA, anti-sense nucleic acid), cellular products such as growth factors (i.e. EGF, HGF, IGF-1, IGF-2, FGF, GDNF, TGF-alpha, TGF-beta, TNF-alpha, VEGF, PDGF and an interleukin).

"Fluidic resistor" refers to a part of the second conduit that resist the flow of fluid back to the pump. The resistor may comprise a section of conduit which is narrowed compared with the first conduit. Thus, when the pump is actuated and fluid is pumped into the actuation chamber, the resistance to flow in the outlet (second) conduit helps cause an increase in pressure in the actuation chamber. The outlet (second) conduit also includes a "Fluidic capacitor" downstream of the fluidic resistor. The purpose of the capacitor is to allow for pressure release in the fluid when the pump is off, and it generally comprises an inflatable chamber that receives fluid and expands to accommodate the fluid pending actuation of the pump.

"Fibrotic encapsulation": Once a device is implanted a complex series of events is initiated to protect the host from the 'foreign body'. Shortly after implantation, fibrinogen and other proteins bind to the surface of the device/foreign body, macrophages then bind and, over time, become multinucleated giant cells releasing inflammatory cytokines. In response to these signals, quiescent fibroblasts are transformed into myofibroblasts, which synthesize procollagen. The procollagen becomes crosslinked and this mature crosslinked collagen and other extracellular matrix proteins contribute to the formation of a dense fibrous capsule that becomes impermeable or hypo-permeable to many compounds.

Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

Materials and Methods

*device of the invention fabrication*

The device manufacture technique for the individual reservoirs is shown in Fig. S4. Briefly, 3 mil (0.075 mm) and 12 mil (0.3 mm) thick thermoplastic urethane TPU sheets (HTM-8001-M and HTM-1001 polyether TPU film, American Polyfilm, Inc) were formed into hemispherical shapes, with a height of 3.9 mm and a diameter of 3.5 mm, using a vacuum thermal former (Yescom Dental Vacuum Former, Generic). 3 mil thick TPU hemispheres were placed inside the 12 mil thick TPU hemispheres and 7 cm of 3 Fr thermoplastic polyurethane catheter tubing (Micro-Renathane MRE037 0.037" x 0.023, Braintree scientific) was bonded to this assembly using a heat transfer machine (Heat Transfer Machine QXAi, Powerpress). This forms the actuation reservoir, which was used for actuating the system with pneumatic pressure via the catheter. A 3 mil thick TPU membrane and a second 9 cm 3 Fr thermoplastic polyurethane catheter was then bonded onto this assembly to form the therapy reservoir which can be used to deliver therapeutics prior to or post implantation via the catheter. This reservoir interfaces with soft tissue via the TPU membrane which can either be nonporous or porous (Fig. 1b) (10 $\mu$m pores, National Centre for Laser Applications, National University of Ireland Galway).

*Implantable system*

A fully implantable stainless steel piezoelectric pump (Dolomite 3200311) was used to demonstrate cyclic actuation of device of the invention. The device was placed in series with a resistive component consisting of narrower diameter tubing (0.2 mm ID, 0.5 mm OD, Polyethylene tubing, SAI Infusions) and a capacitor consisting of a TPE reservoir (2 mm by 1.5 mm, Stretchlon 200, FibreGlast) (Figure S1a). Turning the pump on and off generated cyclic actuation of device of the invention. While the pump was on, the resistive component caused filling of the device reservoir. While the pump was off, fluid flowed from the reservoir to the capacitor and energy was dissipated. Using this circuit, we were able to recapitulate the average peak force achieved with the external control box (Figure S1b). The pump was operated with the pump control board (Dolomite 3200313) and the Mitos Flow Control Centre (FCC) software. The output voltage was +/-80 V, the pulse width was +/- 20 $\mu$s, and the frequency was 350 Hz.

*Experimental characterization of the device of the invention*

ASTM D638 uniaxial tensile tests were performed on a ZwickRoell material testing machine with laser cut TPU specimens. Using the material evaluation feature in Abaqus 2018 (Dassault Systemes, Velizy-Villacoublay, France), four hyperelastic constitutive models (Neo-Hookean, Yeoh, Mooney-Rivlin and Ogden 3rd order) were evaluated. As shown in Fig. 2b, the Ogden 3rd order model had excellent curve fitting performance and therefore was used for the model. The strain energy density function is shown in Fig. S2. The actuation force of the device was measured using an Instron 5944 material testing machine. The set-up is shown in Fig. S2. An acrylic test-rig (Fig. S2) was laser cut to allow the lower membrane of the device to contact the upper compression plate for force measurement. The thickness of the acrylic piece (10 mm) and the diameter of the hollow cylinder (5 mm) were designed to be larger than the height and the diameter of the outer balloon allowing unconstrained deformation of the reservoir. The distance between the compression plates was fixed at the thickness of the acrylic holder. The force was measured using a 50 N load cell. The test the effect of fatigue on the devices the in vivo actuation regimes (Fig. 3b which corresponds to 8400 cycles) were applied to device of the invention in vitro and burst pressure testing was carried out (n=4/group). A piezoelectric pressure sensor (AMS5812, Analog Microelectronics Ltd) with an Arduino UNO interface was connected to the device and syringe via a T-valve. A syringe pump was used to inflate the devices to failure and burst pressure was recorded.

*Computational modelling*

All simulations were conducted in the commercially available software, Abaqus 2018 (Dassault Systemes, Velizy-Villacoublay, France). Since the reservoir is comprised of thin thermoplastic urethane sheets, shell structural analysis was chosen for the simulation. The device was modell
ed as a 3D surface geometry, which contains the outer, middle and lower membrane, as shown in Fig. S2. The appropriate thickness of each shell was assigned. All parts were meshed with 2603 four-node shell elements (S4R). Dirichlet boundary condition were applied to the protruding edge of the lower membrane and all other surfaces were allowed to deform freely. A 2 psi ramp loading was applied to the internal surface of the inner and outer balloons for a duration of 500 ms. These boundary conditions were representative of the in vivo scenario when the reservoir was sutured to the muscular tissue by the edge of the lower membrane and actuated with a cyclic pressure with magnitude of 2 psi and 1 Hz. We also created a fluid structure interaction model using the smooth particle hydrodynamics (SPH) technique to study the effect of actuation on surrounding fluid flow. A $20\times20\times5$ mm3 region was created and filled with 8000 particles. The particles were assigned with a density of 9.96e-7 kg/mm$^3$, bulk modulus of 20.94 MPa and dynamic viscosity of 3.56e-8 MPa.s. Contact pairs were initiated between the shell structure and the particles. Loading and boundary conditions of the shell were the same as the structural analysis.

*In vivo studies*

Animal procedures were reviewed and approved according to ethical regulations by the Institutional Animal Care and Use Committees at Massachusetts Institute of Technology. Female Sprague Dawley rats (225-300 g) were anaesthetized using isoflurane (1-3% isoflurane in oxygen). Animals were treated with a single dose of sustained release buprenorphine (Bup-SR) at 1 mg/kg subcutaneously to control pain. 2 device of the invention reservoirs (porous or non-porous) were implanted subcutaneously in the rat, the device implantation procedure is shown in Fig. 3a. Briefly, the hair on the back of the rat was removed and surgical sites were prepared with 3 washes of Betadine and 70% ethanol. Devices were sterilized using ethylene oxide prior to implantation. An anterior incision was made at the base of the neck for the and 2 posterior incisions were made 9 cm from the original incision along the back of the rat, 1 cm lateral of the spine. A blunt dissection was made at all incisions and a forceps was used to tunnel subcutaneously from the anterior to the posterior sites. A vascular access button, or self-sealing subcutaneous port (VAB95BS-MRI, VABM2B/22R22, VABR4B/22 Instech Laboratories), was connected to the dorsal end of the catheter of each device of the invention reservoir. The port was placed in position at the base of the neck and the devices tunneled posteriorly into position. The port was secured to the underlying fascia using at least one interrupted suture (5-0 monofilament). Each device of the invention reservoir was secured to the underlying fascia with one suture at either side (7-0 monofilament). The skin was closed with interrupted sutures (5-0 monofilament) and the animal was allowed to recover on a heated pad. 3 mL of warm saline were administered subcutaneously. The following day, one of the two implanted device of the invention reservoirs was actuated at controlled input pressures of 1 PSI (Regime 1) or 2 PSI (Regime 2) at 1 Hz every 12 hours for 14 days (Fig. 3b) and compared to the non-actuated control reservoir for analysis. On day 15, animals were sacrificed by CO2. Following sacrifice, each device and the immediate surrounding tissue were extracted. Tissues were fixed for 24 h using 10% Formalin (pH 7.4). The tissue was then washed in 0.2 M phosphate-buffered saline with a final wash in 70% ethanol.

*IVIS imaging*

On days 3, 8 and 14 post-implantation, diffusion of a fluorescent imaging agent (Genhance 750 Fluorescent Imaging Agent, NEV10118, Perkin Elmer) out of the porous device of the invention reservoirs was evaluated using an IVIS Spectrum-bioluminescent and fluorescent imaging system (Perkin Elmer). Images were acquired using a 745 nm/800nm excitation-emission filter pair. Prior to injection of agent, the line was cleared by pulling a vacuum with a syringe connected to the subcutaneous port. The skin above the device of the invention reservoir area was shaved and marked to aid with region of interest (ROI) placement for quantitative analysis. After a background image was acquired, 35 $\mu$L of fluorescent agent were injected through the subcutaneous port either manually or with a syringe-pump (150 $\mu$L/min, Harvard Apparatus). Images were acquired for up to 1 hour following injection ~ every 3 min. An ROI (28.24x28.24 pixels) was placed at the centre of each device of the invention reservoir in every image and total radiant efficiency was measured using the Living Image 3.2 software. Total radiant efficiency measured immediately after injection was subtracted from the subsequent measurements to only account for the change in radiant efficiency due to diffusion over an hour.

*Functional measurements following epinephrine injection*

To illustrate how actuation can improve the time to functional effect after small-molecule delivery, 35 uL of epinephrine (1 mg/ml) were delivered through the system while recording blood pressure with an apically inserted pressure-volume catheter (Millar) as previously described [13].

*microCT, histological and immunohistochemical analysis*

For microCT analysis, fixed tissue samples were transferred to a 2.5% PMA solution in 70% ethanol for 7 days, then washed and stored in fresh 70% ethanol. MicroCT images were captured using a $\mu$CT 100 scanner (Scanco) at 70 kVp and 85 $\mu$A with a 0.5mm Aluminum filter. microCT dicom files were segmented using Mimics Research 18.0.0.525 software as described in Supplementary Material.

Fixed tissue samples (n=3) were transected in half, orientated and embedded in paraffin wax blocks for histological and immunohistochemical analysis. Sections of 5 $\mu$m were cut, deparaffinised in xylene, and rehydrated through a series of graded alcohols. For histological fibrotic capsule quality analysis, sections were stained with 0.1% Picrosirius red/fast green solution (1:1) using an automated stainer Leica ST5010 Autostainer XL. Slides were imaged using Ocularâ2.0 Imaging Software on an Olympus BX41 Microscope with Olympus U-AN360P analyser under a 20x objective lens. Quantification of the collagen content was performed using a previously reported technique [17] and described in Supplementary Material. For immunohistochemical analysis, primary antibodies of CD31 (ab28364, Abcam) (1:200), CD68 (MCA341, Bio Rad) (1:300) and $\alpha$-SMA (ab5694, Abcam) (1:100) were incubated for 1 hour at 37 °C. Secondary antibodies of Alexa Fluor® 594 goat anti-mouse IgG (ThermoFisher Scientific) (1:200), Alexa Fluor® 594 goat anti-rabbit IgG (ThermoFisher Scientific) (1:200) and Alexa Fluor® 488 goat anti-mouse IgG (ThermoFisher Scientific) (1:200) were incubated for 60 min at room temperature, respectively. Sections were stained with Hoechst and coverslipped using fluoromount. Immunofluorescence-stained slides were observed using a spinning disc inverted confocal microscope (Yokagawa CSU22) combined with Andor iQ 2.3 software. Analysis of Macrophages: 20 random fields of view were acquired from 8 sections using confocal microscopy. Using stereological methods an unbiased estimation of area fraction and numerical density (Nv) was calculated [14-16]. Numerical density refers to the number of, in this case macrophages, within a unit volume of tissue. Analysis of Myofibroblasts: 20 random fields of view were acquired from 8 sections using confocal microscopy. Area Fraction was estimated using Image J (Fiji verson 2.0.0) software. This was done by way of auto thresholding using the otsu filter which eliminates autoflouresence and calculates the area fraction of tissue occupied by $\alpha$SMA+ cells. The area fraction was then multiplied by the average volume of c in both control and regime groups to provide an estimate of the total volume of $\alpha$SMA+ cells to assess whether the presence of myfibroblasts were perturbed by actuation.

*Statistical Analysis*

GraphPad Prism (8.1.0) was used for statistical analysis. Normality of distribution was assessed by the Shapiro-Wilk test. Subsequent parametric and/or non-parametric tests were performed. For parametric data, an unpaired t-test was performed for comparing between two groups and a one-way or two-way analysis of variance (ANOVA) with post-hoc Tukey's multiple comparison for comparing between groups. For non-parametric data, a Mann-Whitney U was performed for comparing between two groups and a Kruskal-Wallis test for comparing more than two groups. Statistical significance was accepted when $p < 0.05$. A minimum of two blinded counters were used for fibrous capsule thickness measurements and immunohistochemical analysis.

*MIMICS segmentation and quantification of fibrous capsule thickness*

Dicoms were opened and cropped between suture points on the device. A green mask was added to threshold the field of view. It was then cleared and edited using multiple slice editor to segment the fibrous capsule manually in the sagittal view. The fibrous capsule was mapped every five segments for the entirety of the CT dicom. The fibrous capsule was visually identified by a uniform change in signal intensity at the muscle border. After approximately fifty segments were mapped, interpolation was used to account for gaps between segments. Following completion of the green mask, an additional yellow mask was applied to the dicom files to threshold the black and remove background. A boolean operator was applied (green mask-yellow mask) and subsequently created a third mask (cyan mask). The cyan mask was then edited using multiple slice editor using axial view to ensure consistency between different views. When fibrous capsule was accurately complete in the cyan mask, it was edited in 3D. A circle (2.4 - 2.6 mm diameter) was dropped in center of fibrous capsule. The 3D file was exported as a STL for further analysis using 3-matic Research 10.0.0.212 software. Fix wizard was applied to the files. Smoothing factor of 0.8 was applied. Auto-remesh was then applied. A wall thickness analysis was generated and data was analysed.

*Quantification of fibrous capsule quality*

Fibrotic capsule quality was assessed using picrosirius red and stained in 0.1% fast green (pH 7, Fast Green FCF; Sigma Aldrich) and 0.1% Sirius red in saturated picric acid (picrosirius red stain), both in the same solution at a 1:1 ratio for 1 hour according to previously established protocols [17]. Following this, the sections were rapidly dehydrated through graded ethanol washes (70-100% v/v). Staining was performed using a Leica ST5010 Autostainer XL (Leica Biosystems; Wetzlar, Germany). The slides were then mounted using DPX mountant (Sigma Aldrich) and left to dry horizontally for five hours. Polarised light micrographs were captured using an Olympus BX4 polarised light microscope (Mason Technology Ltd. Dublin, Ireland) at 20x magnification. The polarising lenses were positioned on the light path before the sample and the second polariser (analyser) after the sample. Images were taken whereby maximum polarisation was achieved by adjustment of the polarising filters, and again orthogonal to this maximum polarisation. The two captured images were merged using the MAX function in ImageJ software (freely available from https://imagej.nih.gov/) which enables a complete visualisation of the collagen fibres present [18]. The total area covered by collagen was first calculated in number of pixels using ImageJ. This was achieved excluding the dark background of the images and with colour thresholding, selecting consistent hue ranges to quantify the areas of collagen that polarised red, orange, yellow or green within each pixel. The recorded data was input into the following equations:

$$\% \text{ Red Fibres} = (\text{Red Pixels})/(\text{Total Collagen Pixels x } 0.01)$$

$$\% \text{ Orange Fibres} = (\text{Orange Pixels})/(\text{Total Collagen Pixels x } 0.01)$$

$$\% \text{ Yelow Fibres} = (\text{Yellow Pixels})/(\text{Total Collagen Pixels x } 0.01)$$

$$\% \text{ Green Fibres} = (\text{Green Pixels})/(\text{Total Collagen Pixels x } 0.01)$$

The isotropy of the polarised collagen fibres was evaluated using the OrientationJ plugin on ImageJ. This method can characterize the orientation and isotropy properties of a region of interest (ROI) in an image, based on the evaluation of the gradient structure tensor and in particular analyse collagen orientation (direction) and the degree to which a dominant direction exists in a tissue sample [19]. The coherency indicates if the local image features are oriented or not: C is 1 when the local structure has one dominant orientation and C is 0 if the image is essentially isotropic in the local neighbourhood.

*Quantitative analysis of blood vessels*

Immunohistochemical staining was performed with CD31 (ab28364, Abcam) (1:200). Slides were incubated for 1 hour at 37 °C. A mouse and rabbit specific HRP/DAB kit (ABC) detection IHC kit (ab64264, Abcam) was used. Sections were counterstained with haematoxylin, dehydrated through a series of graded alcohols, cleared in xylene and coverslipped using DPX. Slides were imaged using an Olympus SlideScanner VS120 with an UPLSAPO 20x objective lens. Analysis of neo-vascularisation: Number per area (Na) and radial diffusion distance (Rd) were all calculated using an unbiased counting frame [15, 16]. This counting frame consists of red forbidden lines and green acceptance lines. Any blood vessel that is cut by a forbidden line is not counted. Blood vessels that appear outside the counting frame or are cut by the acceptance lines without also cutting the forbidden line are counted. The application of this counting formula generates an unbiased estimate of the number of blood vessels per unit area. $Na = cN/(cPts \times \text{Area of Grid})$

taking cN as the cumulative no.of blood vessels counted and cPts as the cumulative no. of points. The length density (Lv) of a blood vessel is the length of blood vessels present per unit volume and is calculated by multiplying the numerical density by 2. From length density the radial diffusion distance (Rd) can be calculated by the following formulas: $Lv=Na \times 2$, $Rd=1/\sqrt{(\pi(Iv))}$

Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

References

1. Lotti, F., et al., Invasive Intraneural Interfaces: Foreign Body Reaction Issues. Frontiers in neuroscience, 2017. 11: p. 497-497.

2. Kozai, T.D.Y., et al., Brain tissue responses to neural implants impact signal sensitivity and intervention strategies. ACS chemical neuroscience, 2014. 6(1): p. 48-67.

3. Cetin, N., et al., Foreign Body Reaction to Dialysis Chatheter and Peritoneal Fluid Eosinophilia in a Child on Continuous Ambulatory Peritoneal Dialysis. Iran J Kidney Dis, 2017. 11(4): p. 319-321.

4. Liu, X., et al., Comparison of the postoperative incidence rate of capsular contracture among different breast implants: a cumulative meta-analysis. PloS one, 2015. 10(2): p. e0116071.

5. Tarakji, K.G., et al., Cardiac implantable electronic device infection in patients at risk. Arrhythmia & electrophysiology review, 2016. 5(1): p. 65.

6. Sharkawy, A.A., et al., Engineering the tissue which encapsulates subcutaneous implants. I. Diffusion properties. Journal of Biomedical Materials Research Part A, 1997. 37(3): p. 401-412.

7. Novak, M.T., F. Yuan, and W.M. Reichert, Modeling the relative impact of capsular tissue effects on implanted glucose sensor time lag and signal attenuation. Analytical and bioanalytical chemistry, 2010. 398(4): p. 1695-1705.

8. Thome-Duret, V., et al., Modification of the sensitivity of glucose sensor implanted into subcutaneous tissue. Diabetes & Metabolism, 1996. 22(3): p. 174-178.

9. Fritschy, W.M., et al., The capsular overgrowth on microencapsulated pancreatic islet graft in streptozotocin and autoimmune diabetic rats. Transplant international, 1994. 7(4): p. 264-271.

10. Soon-Shiong, P., et al. An immunologic basis for the fibrotic reaction to implanted microcapsules. in Transplantation proceedings. 1991.

11. Sharkawy, A.A., et al., Engineering the tissue which encapsulates subcutaneous implants. III. Effective tissue response times. Journal of Biomedical Materials Research Part A, 1998. 40(4): p. 598-605.

12. Sharkawy, A.A., et al., Engineering the tissue which encapsulates subcutaneous implants. II. Plasma-tissue exchange properties. Journal of Biomedical Materials Research Part A, 1998. 40(4): p. 586-597.

13. Whyte, W., et al., Sustained release of targeted cardiac therapy with a replenishable implanted epicardial reservoir. Nature Biomedical Engineering, 2018.

14. Howard, C. and M. Reed, Unbiased Stereology, 2nd Edn. Liverpool. 2010, QTP Publications.

15. Jensen, E. and H. Gundersen, The stereological estimation of moments of particle volume. Journal of applied probability, 1985. 22(1): p. 82-98.

16. Dockery, P. and J. Fraher, The quantification of vascular beds: a stereological approach. Experimental and molecular pathology, 2007. 82(2): p. 110-120.

17. Monaghan, M.G., et al., Exogenous miR-29B Delivery Through a Hyaluronan-Based Injectable System Yields Functional Maintenance of the Infarcted Myocardium. Tissue Eng Part A, 2018. 24(1-2): p. 57-67.

18. Coleman, R., Picrosirius red staining revisited. Acta Histochem, 2011. 113(3): p. 231-3.

19. Rezakhaniha, R., et al., Experimental investigation of collagen waviness and orientation in the arterial adventitia using confocal laser scanning microscopy. Biomechanics and Modeling in Mechanobiology, 2012. 11(3): p. 461-473.

20. Roche, E.T., et al., Soft robotic sleeve supports heart function. Science translational medicine, 2017. 9(373): p. eaaf3925.

21. Horvath, M.A., et al., An Intracardiac Soft Robotic Device for Augmentation of Blood Ejection from the Failing Right Ventricle. Annals of Biomedical Engineering, 2017. 45(9): p. 2222-2233.

**Claims**

1. An implantable medical device having a soft tissue interfacing surface comprising at least one actuatable capsule having a soft tissue interfacing deflectable membrane configured for cyclical deflection upon actuation of the capsule to modulate the biomechanics of the soft tissue interface during use.

2. An implantable medical device according to Claim 1, in which the actuatable capsule is configured for pneumatic, hydraulic, electrical, or ultrasound actuation.

3. An implantable medical device according to Claim 2, in which the at least one actuatable capsule comprises:

   an actuation chamber containing a first fluid;
   a therapeutic chamber containing a second fluid;
   a deflectable membrane separating the actuation chamber and therapeutic chamber; and
   an actuation conduit in fluidic communication with the actuation chamber for pneumatic actuation of the actuation chamber,
   wherein the therapeutic chamber comprises the soft tissue interfacing deflectable membrane.

4. An implantable medical device according to any preceding Claim, in which the at least one actuatable capsule is a millisized capsule.

5. An implantable medical device according to any preceding Claim, in which the tissue interfacing surface comprises an array of actuatable capsules.

6. An implantable medical device according to any preceding Claim, in which the soft tissue interfacing surface of the medical device comprises a cover or sheath configured for retrofitting to the medical device, and in which the soft tissue interfacing deflectable membrane is disposed on the soft tissue interfacing surface with the soft tissue interfacing deflectable membrane substantially flush with the surface.

7. An implantable medical device according to any preceding Claim, in which the or each capsule is dome shaped having a flat base provided by the soft tissue interfacing deflectable membrane.

8. An implantable medical device according to any of Claims 2 to 8, in which the or each capsule comprises a therapeutic conduit in fluidic communication with the therapeutic chamber for delivery of an active agent, and in which the soft tissue interfacing deflectable membrane is porous or configured to become porous upon actuation/deflection.

9. An implantable medical device according to any of Claims 2 to 9, in which the or each capsule comprises a second actuation conduit in fluidic communication with the actuation chamber.

10. An implantable medical device according to any of Claims 2 to 9, including an implantable pump operatively connected to the first and/or second actuation conduit.

11. An implantable medical device according to Claim 10, in which the pump is operatively connected to the first and second conduits in series and is configured to pump fluid to the first conduit and receive fluid from the other conduit.

12. An implantable medical device according to Claim 11, in which the second conduit comprises a fluidic resistor configured to resist fluid flow and an expandable chamber distal of the fluidic resistor

13. An implantable medical device according to any preceding Claim, selected from a tissue implant, an indwelling catheter, a pacemaker, a biosensor, a drug or cell delivery device, and a neural probe.

14. A retro-fit skin for an implantable medical device configured to retro-fit to the medical device and adhere to and cover at least part of a soft tissue interfacing surface of the medical device, the skin comprising at least one actuatable capsule having a soft tissue interfacing deflectable membrane configured for deflection upon actuation of the capsule to modulate the biomechanics of the soft tissue interface during use.

15. A retro-fit skin according to Claim 14, in which the actuatable capsule comprises:

an actuation chamber containing a first fluid;
a therapeutic chamber containing a second fluid;
a deflectable membrane separating the actuation chamber and therapeutic chamber; and
an actuation conduit in fluidic communication with the actuation chamber for pneumatic actuation of the actuation chamber,
wherein the therapeutic chamber comprises a soft tissue interfacing deflectable membrane.

**FIGURE 1A**

**OPTION A** • **NON POROUS** • For Surface Modification

1. OUTER BALLOON 12 mil TPU
2. INNER BALLOON 3mil TPU
3. MEMBRANE 3 mil TPU
4. CATHETER

ACTUATION CATHETER

EXPLODED VIEW

Pressure = atm

Actuation Catheter
Actuation Reservoir
Middle Membrane
Outer Membrane
Therapy Reservoir
Membrane
HOST TISSUE
Suture

flat membrane

Pressure > atm

convex membrane

**FIGURE 1B**

OPTION B • POROUS • For Therapy Delivery

**EXPLODED VIEW**

1. OUTER BALLOON 12 mil TPU
2. INNER BALLOON 3mil TPU
3. MEMBRANE 3 mil TPU
4. CATHETER

THERAPY DELIVERY CATHETER

ACTUATION CATHETER

Pressure = atm

Therapy Delivery Catheter
Actuation Reservoir
Middle Membrane
Outer Membrane
Therapy Reservoir
Actuation Catheter
Membrane
Suture

**HOST TISSUE**

flat membrane

Pressure > atm — Actuation
FLUID
convex membrane

Pressure = atm — Therapy delivery
THERAPY
flat membrane

## FIGURE 1C

EP 3 769 719 A1

**FIGURE 2A**

**FIGURE 2B**

Mises Stress (MPa)

**FIGURE 2C**

Max In-Plane Strain

**FIGURE 2D**

**FIGURE 2E**

**FIGURE 2F**

**FIGURE 2G**

**FIGURE 2H**

EP 3 769 719 A1

**1** MAKE 1 CM INCISION AT BASE OF NECK BETWEEN SHOULDER BLADES

**2** MEASURE 7 CM FROM INCISION ALONG BACK OF ANIMAL, 1 CM EITHERSIDE OF THE SPINE

**3** MEASURE 9CM FROM INCISION ALONG BACK OF ANIMAL, 1 CM EITHER SIDE OF THE SPINE

**4** MAKE THE PLACEMENT INCISION ON THE RIGHTSIDE OF THE ANIMAL TO CREATE THE SUBCUTANEOUS POCKET FOR DEVICE 1

**5** MAKE A BLUNT DISSECTION

**6** REPEAT STEPS 4 & 5 ON THE LEFT SIDE

**7** PLACE SUBCUTANEOUS PORT IN POSITION AT BASE OF NECK AND FEED THE DEVICES THROUGH THE SUBCUTANEOUS TUNNELS

**8** PLACE A SUTURE THROUGH EACH SIDE OF THE SUBCUTANEOUS PORT AND UNDERLYING MUSCLE TO SECURE

**9** PLACE EXPANDER IN THE RIGHT INCISION TO MAKE THE DEVICE RESERVOIR CLEARLY VISIBLE. BRING A SUTURE THROUGH THE SUTURE HOLE AND UNDERLYING MUSCLE TO SECURE IN PLACE

**10** REPEAT STEP 9 ON THE LEFT

**11** CLOSE THE PLACEMENT INCISION WITH INTERRUPTED BRAIDED SUTURE EACH SIDE OF THE PORT

**12** CLOSE THE SUBCUTANEOUS PORT INCISIONS WITH BRAIDED SUTURES

**FIGURE 3A**

EP 3 769 719 A1

| Experimental group | Experimental timeline |
|---|---|
| Non-porous (- actuation) n=6 | |
| Non-porous (+ actuation) n=6 | Actuation – regime 1 or 2 |
| Porous (- actuation) n=3 | |
| Porous (+ actuation) n=3 | Actuation regime 2 |

D-1  D0  D3  D8  D14

Implant  IVIS  IVIS  IVIS

D15

Adrenaline
delivery

**Post-sacrifice**

microCT

Histology

Immunohistochemistry

Actuation:

D0-D14: 1 Hz for 5 mins every 12H

Regime 1: 1psi input pressure

Regime 2: 2 psi input pressure

## FIGURE 3B

**FIGURE 4A**

0.2 mm

0 mm

Regime 2

Regime 1

Control

**FIGURE 4B**

**FIGURE 4C**

**A)**

**FIGURE 5A**

**B)**

**FIGURE 5B**

**C)**

**FIGURE 5C**

**D)**

**F)**

# FIGURE 5D AND 5F

**E)**

# FIGURE 5E

FIGURE 5G

## FIGURE 6

| Circular Arrays | Linear/Cylindrical Arrays |
|---|---|
| Model | Model |
| Proof of Concept Prototype | Proof of Concept Prototype |

Device Application
- Breast Implants -

Device Application
- Implantable Pacemaker Leads -

Clinical Problem
- Capsular Contracture

Clinical Problem
- Fibrosis at tip

**FIGURE 7A**

**FIGURE 7B**

**FIGURE 7C**

**FIGURE 7D**

**FIGURE 8A**

**FIGURE 8B**

**FIGURE 9A**

Electropneumatics and control

# FIGURE 9B

# FIGURE 9C

**FIGURE 10A**

**FIGURE 10B**

$$U = \sum_{i=1}^{N} \frac{2\mu_i}{\alpha_i^2} \left( \bar{\lambda}_1^{\alpha_i} + \bar{\lambda}_2^{\alpha_i} + \bar{\lambda}_3^{\alpha_i} - 3 \right) + \sum_{i=1}^{N} \frac{1}{D_i} \left( J^{el} - 1 \right)^{2i},$$

**FIGURE 10C**

**FIGURE 10D**

**FIGURE 10E**

S, Mises
SNEG, (fraction = -1.0)
(Avg: 75%)

2.086
1.800
1.650
1.500
1.350
1.200
1.050
0.900
0.750
0.600
0.450
0.300
0.150
0.000

Porous membrane

S, Mises
SNEG, (fraction = -1.0)
(Avg: 75%)

1.800
1.650
1.500
1.350
1.200
1.050
0.900
0.750
0.600
0.450
0.300
0.150
0.000

Non-porous membrane

**FIGURE 10F**

LE, Max. In-Plane Principal
SNEG, (fraction = -1.0)
(Avg: 75%)
- 0.064
- 0.059
- 0.053
- 0.048
- 0.043
- 0.037
- 0.032
- 0.027
- 0.021
- 0.016
- 0.011
- 0.005
- 0.000

Y
X
Z

Non-porous membrane

LE, Max. In-Plane Principal
SNEG, (fraction = -1.0)
(Avg: 75%)
- 0.086
- 0.064
- 0.059
- 0.053
- 0.048
- 0.043
- 0.037
- 0.032
- 0.027
- 0.021
- 0.016
- 0.011
- 0.005
- 0.000

Y
X
Z

Porous membrane

**FIGURE 10G**

EP 3 769 719 A1

# BALLOON THERMOFORMING

## Equipment

THERMOFORMER

POSITIVE MOULD

## Process

**(1)**
TPU
place TPU sheet

**(2)**
Secure in place

**(3)**
bring TPU up

**(4)**
turn on Heat

**(5)**
HEAT
heat the TPU

**(6)**
HEAT
bring heated TPU over mould

**(7)**
AIR
Turn on Vacuum

Take mould out of thermoformer

## FIGURE 11A

# HEAT SEALING

## Equipment

HEAT PRESS

NEGATIVE MOULD

## Process

(1) Cut both 12 mil and 3 mil positive molds

(2) place 12 mil outer balloon in negative mould first

(3) place 3 mil inner balloon in negative mould

(4) place sheet of teflon on top

(5) bring down heat arm of heat press

(6) heat for 13 seconds at 330 F/160 C

(7) Remove Heat and Teflon

(8) Completed Device

## FIGURE 11B

Europäisches
Patentamt

European
Patent Office

Office européen
des brevets

# EUROPEAN SEARCH REPORT

Application Number

EP 19 18 7842

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 718 893 A (DORMAN FRANK D [US] ET AL) 12 January 1988 (1988-01-12) | 1-7, 10-15 | INV. A61F2/12 A61M5/142 |
| A | * the whole document * | 8 | |
| X | US 5 707 361 A (SLETTENMARK BRUNO [SE]) 13 January 1998 (1998-01-13) * the whole document * | 1,14 | |
| X | US 9 724 189 B2 (FORSELL PETER [CH]) 8 August 2017 (2017-08-08) * the whole document * | 1 | |
| A | US 2014/371674 A1 (WANG BIN [US] ET AL) 18 December 2014 (2014-12-18) * figure 5 * | 1-15 | |
| A | US 2009/177157 A1 (VAN BRUGGEN MICHEL PAUL BARBAR [NL] ET AL) 9 July 2009 (2009-07-09) * paragraphs [0007], [0054]; figures 1-5 * | 1-15 | |
| A | US 2006/069403 A1 (SHALON TADMOR [US] ET AL) 30 March 2006 (2006-03-30) * figures 7A, B * | 1-15 | TECHNICAL FIELDS SEARCHED (IPC) A61F A61M |
| A | EP 2 900 212 A1 (PALO ALTO RES CT INC [US]) 5 August 2015 (2015-08-05) * figure 9 * | 1-15 | |
| A | US 2015/126968 A1 (ABHISHEK RAMKUMAR [US] ET AL) 7 May 2015 (2015-05-07) * figures 1-5c * | 1-15 | |

The present search report has been drawn up for all claims

| Place of search | Date of completion of the search | Examiner |
|---|---|---|
| Munich | 4 October 2019 | Haslinde, Verena |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 19 18 7842

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-2019

| Patent document cited in search report | | Publication date | Patent family member(s) | | Publication date |
|---|---|---|---|---|---|
| US 4718893 | A | 12-01-1988 | AU | 594269 B2 | 01-03-1990 |
| | | | CA | 1262325 A | 17-10-1989 |
| | | | DK | 521287 A | 04-08-1988 |
| | | | EP | 0291510 A1 | 23-11-1988 |
| | | | IL | 81444 A | 18-07-1991 |
| | | | JP | S63503359 A | 08-12-1988 |
| | | | US | 4718893 A | 12-01-1988 |
| | | | WO | 8704629 A1 | 13-08-1987 |
| US 5707361 | A | 13-01-1998 | FR | 2717084 A1 | 15-09-1995 |
| | | | JP | H07255843 A | 09-10-1995 |
| | | | US | 5707361 A | 13-01-1998 |
| US 9724189 | B2 | 08-08-2017 | AU | 2010272575 A1 | 08-03-2012 |
| | | | BR | 112012001029 A2 | 22-11-2016 |
| | | | CA | 2805679 A1 | 20-01-2011 |
| | | | EP | 2453839 A1 | 23-05-2012 |
| | | | US | 2012116509 A1 | 10-05-2012 |
| | | | US | 2017333179 A1 | 23-11-2017 |
| | | | WO | 2011006900 A1 | 20-01-2011 |
| US 2014371674 | A1 | 18-12-2014 | AU | 2014281685 A1 | 21-01-2016 |
| | | | CA | 2915378 A1 | 24-12-2014 |
| | | | CN | 105555334 A | 04-05-2016 |
| | | | EP | 3010567 A1 | 27-04-2016 |
| | | | JP | 2016526403 A | 05-09-2016 |
| | | | US | 2014371674 A1 | 18-12-2014 |
| | | | WO | 2014204957 A1 | 24-12-2014 |
| US 2009177157 | A1 | 09-07-2009 | CN | 101432033 A | 13-05-2009 |
| | | | EP | 2015801 A2 | 21-01-2009 |
| | | | JP | 2009535083 A | 01-10-2009 |
| | | | US | 2009177157 A1 | 09-07-2009 |
| | | | WO | 2007125456 A2 | 08-11-2007 |
| US 2006069403 | A1 | 30-03-2006 | AU | 2005286840 A1 | 30-03-2006 |
| | | | CA | 2581320 A1 | 30-03-2006 |
| | | | DK | 1811914 T3 | 28-09-2015 |
| | | | EP | 1811914 A2 | 01-08-2007 |
| | | | ES | 2547448 T3 | 06-10-2015 |
| | | | JP | 5009158 B2 | 22-08-2012 |
| | | | JP | 2008513182 A | 01-05-2008 |
| | | | US | 2006069403 A1 | 30-03-2006 |
| | | | US | 2010010531 A1 | 14-01-2010 |
| | | | WO | 2006034273 A2 | 30-03-2006 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 19 18 7842

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-2019

| Patent document cited in search report | | Publication date | Patent family member(s) | | Publication date |
|---|---|---|---|---|---|
| EP 2900212 | A1 | 05-08-2015 | AR | 092582 A1 | 29-04-2015 |
| | | | AU | 2013324282 A1 | 09-04-2015 |
| | | | BR | 112015006804 A2 | 04-07-2017 |
| | | | CA | 2885891 A1 | 03-04-2014 |
| | | | CN | 104812371 A | 29-07-2015 |
| | | | CN | 105769305 A | 20-07-2016 |
| | | | EP | 2900212 A1 | 05-08-2015 |
| | | | JP | 6224716 B2 | 01-11-2017 |
| | | | JP | 2015536694 A | 24-12-2015 |
| | | | NZ | 706319 A | 27-10-2017 |
| | | | RU | 2015113731 A | 20-11-2016 |
| | | | US | 2014088346 A1 | 27-03-2014 |
| | | | US | 2015105610 A1 | 16-04-2015 |
| | | | US | 2015105751 A1 | 16-04-2015 |
| | | | WO | 2014051835 A1 | 03-04-2014 |
| US 2015126968 | A1 | 07-05-2015 | BR | 112016010062 A2 | 20-03-2018 |
| | | | EP | 3065799 A1 | 14-09-2016 |
| | | | US | 2015126968 A1 | 07-05-2015 |
| | | | WO | 2015069700 A1 | 14-05-2015 |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LOTTI, F. et al.** *Invasive Intraneural Interfaces: Foreign Body Reaction Issues. Frontiers in neuroscience,* 2017, vol. 11, 497-497
- **KOZAI, T.D.Y. et al.** Brain tissue responses to neural implants impact signal sensitivity and intervention strategies. *ACS chemical neuroscience,* 2014, vol. 6 (1), 48-67
- **CETIN, N. et al.** Foreign Body Reaction to Dialysis Chatheter and Peritoneal Fluid Eosinophilia in a Child on Continuous Ambulatory Peritoneal Dialysis. *Iran J Kidney Dis,* 2017, vol. 11 (4), 319-321
- **LIU, X. et al.** Comparison of the postoperative incidence rate of capsular contracture among different breast implants: a cumulative meta-analysis. *PloS one,* 2015
- **TARAKJI, K.G. et al.** Cardiac implantable electronic device infection in patients at risk. *Arrhythmia & electrophysiology review,* 2016, vol. 5 (1), 65
- **SHARKAWY, A.A. et al.** Engineering the tissue which encapsulates subcutaneous implants. I. Diffusion properties. *Journal of Biomedical Materials Research Part A,* 1997, vol. 37 (3), 401-412
- **NOVAK, M.T. ; F. YUAN ; W.M. REICHERT.** Modeling the relative impact of capsular tissue effects on implanted glucose sensor time lag and signal attenuation. *Analytical and bioanalytical chemistry,* 2010, vol. 398 (4), 1695-1705
- **THOME-DURET, V. et al.** Modification of the sensitivity of glucose sensor implanted into subcutaneous tissue. *Diabetes & Metabolism,* 1996, vol. 22 (3), 174-178
- **FRITSCHY, W.M. et al.** The capsular overgrowth on microencapsulated pancreatic islet graft in streptozotocin and autoimmune diabetic rats. *Transplant international,* 1994, vol. 7 (4), 264-271
- **SOON-SHIONG, P. et al.** An immunologic basis for the fibrotic reaction to implanted microcapsules. *Transplantation proceedings,* 1991

- **SHARKAWY, A.A. et al.** Engineering the tissue which encapsulates subcutaneous implants. III. Effective tissue response times. *Journal of Biomedical Materials Research Part A,* 1998, vol. 40 (4), 598-605
- **SHARKAWY, A.A. et al.** Engineering the tissue which encapsulates subcutaneous implants. II. Plasma-tissue exchange properties. *Journal of Biomedical Materials Research Part A,* 1998, vol. 40 (4), 586-597
- **WHYTE, W. et al.** Sustained release of targeted cardiac therapy with a replenishable implanted epicardial reservoir. *Nature Biomedical Engineering,* 2018
- **HOWARD, C. ; M. REED.** Unbiased Stereology, 2nd Edn. QTP Publications, 2010
- **JENSEN, E. ; H. GUNDERSEN.** The stereological estimation of moments of particle volume. *Journal of applied probability,* 1985, vol. 22 (1), 82-98
- **DOCKERY, P. ; J. FRAHER.** The quantification of vascular beds: a stereological approach. *Experimental and molecular pathology,* 2007, vol. 82 (2), 110-120
- **MONAGHAN, M.G. et al.** Exogenous miR-29B Delivery Through a Hyaluronan-Based Injectable System Yields Functional Maintenance of the Infarcted Myocardium. *Tissue Eng Part A,* 2018, vol. 24 (1-2), 57-67
- **COLEMAN, R.** Picrosirius red staining revisited. *Acta Histochem,* 2011, vol. 113 (3), 231-3
- **REZAKHANIHA, R. et al.** Experimental investigation of collagen waviness and orientation in the arterial adventitia using confocal laser scanning microscopy. *Biomechanics and Modeling in Mechanobiology,* 2012, vol. 11 (3), 461-473
- **ROCHE, E.T. et al.** Soft robotic sleeve supports heart function. *Science translational medicine,* 2017, vol. 9 (373), 3925
- **HORVATH, M.A. et al.** An Intracardiac Soft Robotic Device for Augmentation of Blood Ejection from the Failing Right Ventricle. *Annals of Biomedical Engineering,* 2017, vol. 45 (9), 2222-2233